# FASCICULE DE BREVET EUROPEEN

(11) **EP 0 576 649 B1**
(45) Date de publication et mention de la délivrance du brevet: **03.04.1996**
(21) Numéro de dépôt: 93901581.4
(22) Date de dépôt: 21.01.1993
(51) Int. Cl.: A61J 1/00, A61F 9/00

(54) **DISPOSITIF DE STOCKAGE D'UNE SUBSTANCE MEDICAMENTEUSE LIQUIDE ET DE DISTRIBUTION DE GOUTTES OPHTALMIQUES**
VORRICHTUNG ZUM AUFBEWAHREN VON FLÜSSIGER MEDIZINSUBSTANZ UND ZUR AUSGABE VON AUGENTROPFEN
DEVICE FOR STORING A LIQUID MEDICINAL SUBSTANCE AND DISPENSING EYE DROPS

(30) Priorité: 21.01.1992 FR 9200728; 09.06.1992 FR 9207167
(43) Date de publication de la demande: 05.01.1994
(73) Titulaire: MEDICORP HOLDING S.A., L-2014 Luxembourg (LU)
(72) Inventeur: Meyer, Gabriel, 1195 Dully (CH)
(74) Mandataire: Nithardt, Roland
(86) Numéro de dépôt international: CH9300014
(87) Numéro de publication internationale: WO9313737

(56) Documents cités:
- EP-A- 0 207 544
- EP-A- 0 439 999
- WO-A-91/16868
- GB-A- 2 010 681
- US-A- 3 934 585
- DESIGN ENGINEERING. Août 1990, LONDON GB 'eye dispenser for glaucoma treatment'

## Description

La présente invention concerne un dispositif de stockage d'une substance médicamenteuse liquide et de distribution de gouttes ophtalmiques constituées par cette substance, comportant un réservoir cylindrique contenant ladite substance et un distributeur adapté à une extrémité de ce réservoir, ce distributeur étant pourvu d'un embout applicateur équipé d'un orifice pour former et déposer lesdites gouttes dans l'oeil d'un patient.

On connaît déjà de nombreux dispositifs de ce type qui ont tous pour objet de stocker et de distribuer une substance médicamenteuse destinée à être déposée sous forme de gouttes dans les yeux d'un patient. Dans la plupart de ces réalisations, le réservoir comporte des parois en matière synthétique souple ou se présente sous la forme d'un soufflet à parois plissées pour permettre à l'utilisateur d'appliquer une pression qui engendre l'évacuation du liquide pour former lesdites gouttes.

Lorsque cette pression est relâchée, le réservoir élastique déformable génère une aspiration au niveau de l'embout applicateur, ce qui a pour effet de réaspirer à l'intérieur du réservoir la dernière goutte qui s'était formée à l'extrémité dudit embout.

Or la contamination bactérienne de l'embout applicateur au moment de l'administration des gouttes est quasiment inévitable en raison de la proximité de cet embout avec l'oeil, la paupière ou les cils. Le liquide qui se trouve à proximité immédiate de l'extrémité de l'embout, et en particulier la dernière goutte qui s'y forme est obligatoirement privilégiée par cette contamination. En outre le milieu humide est particulièrement favorable à la croissance des germes dont la migration vers l'intérieur du réservoir ne peut être empêchée. Cette migration est accélérée lorsque la dernière goutte est réaspirée dans l'embout.

Pour éviter que l'ensemble de la substance de traitement contenue dans le réservoir ne soit contaminée, et pour enrayer la progression de la contamination bactérienne, on ajoute à cette substance des agents conservateurs appropriés.

Toutefois, il est à noter que l'effet bactéricide des agents conservateurs est limité dans le temps. Pour cette raison les solutions de traitement utilisées comme gouttes ophtalmiques ne sont réputés stériles que pendant une durée de l'ordre de trente jours à partir de la première utilisation. Cette contrainte provoque des pertes économiques et génère des risques d'infection de l'oeil chez les patients ne respectant pas les consignes d'utilisation.

Les systèmes connus à réservoir souple en matière synthétique ne se prêtent pas à une stérilisation classique par autoclavage en raison des hautes températures que ne supportent pas les matières synthétiques habituellement utilisées. Ce fait constitue une deuxième raison justifiant la présence d'agents conservateurs bactéricides.

Malheureusement, la présence d'agents conservateurs avec des concentrations suffisantes pour qu'ils soient réellement efficaces constitue un inconvénient important parce qu'ils sont la cause de problèmes majeurs pour le patient. Ils peuvent provoquer une dénaturisation de la cornée, des irritations, des allergies, etc.

Afin d'exclure les effets secondaires provoqués par ces agents conservateurs, ceux-ci doivent être éliminés avant que les gouttes soient déposées dans l'oeil du patient.

Un moyen connu consiste à équiper le dispositif d'une membrane, d'un tampon ou d'un filtre absorbant spécifiquement l'agent conservateur au moment de l'administration de la goutte. Un tel moyen est par exemple décrit dans le brevet US-A-5'056'689. Toutefois, de tels moyens absorbants sont difficiles à réaliser pour agir spécifiquement sur les agents conservateurs utilisés sans risque d'agir sur la substance active de traitement.

Les possibilités d'agir efficacement sont limitées et nécessitent des investissements importants pour la recherche de tels moyens et leur mise au point. Le dispositif du brevet américain est équipé d'une soupape unidirectionnelle pour tenter d'éviter la réaspiration directe par l'embout distributeur et ledit embout est équipé d'une autre soupape unidirectionnelle permettant l'admission d'air non stérile à l'intérieur du récipient. La présence de ces deux soupapes ne résout pas le problème de la contamination de l'embout et de la solution. Cette réalisation engendre des coûts supplémentaires s'ajoutant au coût du moyen absorbant et de l'agent conservateur lui-même. Dans la pratique, l'agent conservateur a été maintenu dans cette solution à cause de la réaspiration résultant de la déformation élastique qui n'a pas pu être empêchée, et du fait qu'aucun moyen permettant d'éviter la prolifération des germes dans l'embout applicateur n'a été prévu.

Les mêmes problèmes sont posés par le dispositif décrit par la demande internationale WO-A-91/16868, ce dispositif comportant un récipient souple ayant des parois compressibles. Il présente tous les inconvénients cités ci-dessus et notamment celui de l'aspiration des gouttes en fin d'utilisation, d'où la contamination du médicament.

En effet, lorsque le dispositif comportant un récipient élastiquement déformable est équipé d'un filtre stérilisant qui doit impérativement rester mouillé pour être efficace, il se forme progressivement un vide de plus en plus poussé à l'intérieur du réservoir, vu que la substance évacuée par poussée sur le corps de ce réservoir est partiellement réaspirée en fin d'utilisation, jusqu'au niveau du filtre stérilisant, et empêche la pénétration d'air provenant de l'extérieur. En fin d' utilisation, la poussée sur les parois souples ou plissées du réservoir, nécessaire pour former des gouttes, devient tellement importante que cette utilisation devient très désagréable pour le patient. Par ailleurs, le phénomène de réaspiration n'exclut pas les risques car si l'embout a été contaminé par l'oeil, cette contamination peut se propager vers l'intérieur du dispositif et éventuellement infecter le filtre stérilisant.

Un autre problème qui n'est pas résolu avec les systèmes existants est celui de la régularité et de la précision des gouttes engendrées. On sait qu'une goutte ophtalmique doit avoir un volume inférieur à 30 microlitres pour que l'oeil ne soit pas "inondé". On a d'autre part mesuré la force optimale qui doit être appliquée par l'utilisateur pour qu'il puisse générer une goutte ophtalmique à partir d'un dispositif distributeur. Cette force est équivalente à environ 300 grammes et devrait être constante tout au long de l'utilisation du dispositif multidose. En plus, le dispositif ne devrait pas permettre de former un jet quelle que soit la force appliquée. La plupart des dispositifs connus ne permettent ni de former des gouttes régulières, ni d'appliquer des forces constantes, ce qui aboutit à une perte importante de substance et à des désagréments d' utilisation. Certains dispositifs ont tenté de remédier à ces inconvénients en introduisant une membrane absorbant les agents conservateurs mais les solutions trouvées sont partielles et ne donnent pas satisfaction à tous niveaux. La formation précise d'une goutte sans réaspiration ni formation d'une deuxième goutte non souhaitée ne peut pas être garantie par les solutions proposées, indépendamment de la présence ou non d'un agent conservateur.

On constate que la suppression de tout agent conservateur est impérative pour préserver la santé du patient, et que les moyens utilisés pour absorber les agents conservateurs ne parviennent pas à résoudre les problèmes de contamination de l'embout, ni à distribuer de manière précise et agréable des gouttes avec une force constante. A cela s'ajoute le fait qu'en raison de la réaspiration, certaines solutions ne restent pas homogènes et forment de la mousse, ce qui provoque des variations de dosage inacceptables.

Le cahier des charges d'un dispositif permettant de supprimer totalement toute présence d'agent conservateur dans la solution et dans les gouttes tout en garantissant le maintien de la stérilité de la solution contenue dans le récipient, et de celle restant dans les canaux de l'organe distributeur, impose de préférence la présence des moyens suivants :
- un moyen permettant d'évacuer la solution avec une force constante;
- un moyen permettant de proscrire la possibilité d'engendrer une dépression à l'intérieur du récipient;
- un moyen permettant de maîtriser le débit sans formation de jet;
- un moyen permettant de supprimer la pression résiduelle à l'intérieur du récipient après l'administration précise d'une goutte pour éviter la formation d'une deuxième goutte à l'extérieur de l'embout;
- un moyen permettant d' obturer de manière étanche et garantie sans risque d'erreur, sans provoquer de surpression, ni de refoulement de la solution contenue dans l'embout;
- un moyen permettant de proscrire les turbulences et les mélanges air/solution à l'intérieur de l'embout distributeur pour former des gouttes homogènes et sans formation de mousse;
- un moyen permettant de proscrire la migration des germes vers l'intérieur du récipient; et
- un moyen permettant de supprimer la prolifération des germes à l'intérieur de l'embout distributeur.

Certains de ces moyens pourraient être remplis par des seringues d'injection comme celle décrite par exemple dans les publications GB-A- 2 010 681 et EP-A-0 207 544, mais les solutions proposées ne sont que partielles et ne donnent pas satisfaction à tous les niveaux et n'ont par conséquent aucune chance d'être acceptées en tant que dispositif ophtalmique sans agent conservateur, car seules la présence et la maîtrise des moyens ci-dessus permettent de garantir la distribution de gouttes stériles.

En effet, les seringues d'injection qui seraient équipées d'embouts compte-gouttes et de filtres stérilisants pourraient être capables de former, de manière imprécise et incontrôlée, des gouttes dont la stérilité ne pourrait être garantie en raison de l'absence de la plupart des moyens mentionnés ci-dessus.

Si dans une seringue la fonction "réaspiration" est indispensable pour la remplir et pour tester la présence de sang, cette fonction est absolument proscrite dans les applicateurs de gouttes ophtalmiques afin de maintenir la stérilité à l'intérieur du flacon, lorsque ce dernier est du type multidose.

Un dispositif distributeur de gouttes ophtalmiques est obligatoirement du type multidose, alors qu'une seringue est nécessairement du type monodose pour préserver la stérilité.

Enfin, il existe des filtres stérilisants à membrane que l'on peut coupler à des seringues d'injection. De tels filtres peuvent accessoirement jouer le rôle de régulateurs de débit et de barrières aseptiques au reflux empêchant la contamination du contenu de la seringue. En modifiant le couplage entre le piston de la seringue et le poussoir afin de supprimer le phénomène de réaspiration dont les inconvénients ont été mentionnés ci-dessus il serait possible, avec le dispositif obtenu de distribuer des gouttes une à une sans formation de jet et sans risque de contamination de l'intérieur. Malgré cette modification majeure des fonctions essentielles d'une seringue qui devrait par ailleurs être équipée d'un embout de distribution de gouttes, il ne serait pas possible de distribuer des gouttes avec précision en raison de la pression résiduelle, ni de maîtriser le risque de contamination et de croissance bactérienne en aval du filtre entre deux applications. Cette observation est valable également pour tous les dispositifs de l'art antérieur, même ceux équipés de moyens permettant d'absorber des agents conservateurs contenus dans la substance médicamenteuse, car la contamination de l'embout est inévitable et le risque de croissance bactérienne en aval des moyens d'absorbtion des agents conservateurs ne peut pas être évité.

D'autre part, la pression résiduelle non maîtrisée provoque des écoulements indésirables dès que l'on cesse d'exercer une pression sur le piston, alors que l'écoulement doit cesser immédiatement pour séparer la goutte avec précision. Cela ne peut pas être réalisé avec un piston classique.

La présente invention se propose de pallier l'ensemble des inconvénients précités et offre un dispositif satisfaisant aux exigences stipulées dans le cahier des charges ci-dessus et aux exigences les plus récentes, tout en simplifiant la fabrication et en facilitant l'utilisation par le patient.

Dans ce but, le dispositif selon l'invention est caractérisé en ce que le réservoir est rigide et obturé, à l'extrémité sur laquelle est adapté le distributeur, par un dispositif de régulation du débit comportant un organe régulateur dont l'ouverture est fonction d'une pression de seuil appliquée à ladite substance médicamenteuse liquide, en ce que ce réservoir est obturé à son autre extrémité par un piston coulissant engagé dans ledit réservoir, ledit piston étant agencé pour générer ladite pression de seuil, en ce que l'organe régulateur de débit est associé à des moyens d'aseptisation ayant une action oligodynamique sur un volume prédéterminé de substance médicamenteuse localisée en amont dudit orifice, et en ce que ledit piston est pourvu de moyens élastiques agencés pour absorber la pression résiduelle.

Suivant les formes de réalisation, le dispositif régulateur de débit peut comporter au moins une membrane à micropores calibrés, au moins un orifice calibré ou au moins un canal étranglé.

De préférence, le réservoir comporte en outre un moyen d'obturation complémentaire amovible agencé pour empêcher le contact de la substance médicamenteuse avec le dispositif régulateur de débit pendant le stockage de cette substance.

Ce moyen d'obturation complémentaire amovible peut être un bouchon d'obturation amovible et le réservoir peut comporter une chambre de dérivation agencée pour loger au moins partiellement ce bouchon pendant l'utilisation de la substance médicamenteuse.

Dans une forme de réalisation avantageuse, le bouchon d'obturation amovible comporte au moins un bourrelet annulaire pour assurer l'étanchéité du réservoir pendant le stockage et au moins un évidement latéral pour assurer l'écoulement de la substance médicamenteuse pendant l'utilisation, cet évidement étant agencé pour communiquer avec au moins un évidement ménagé à la base du réservoir.

Suivant la forme de réalisation, la hauteur de la chambre de dérivation peut être inférieure ou supérieure à la hauteur du bouchon d'obturation amovible.

De préférence, le bouchon d'obturation amovible comporte une zone centrale d'épaisseur réduite et le réservoir est équipé d'une aiguille agencé pour percer ce bouchon dans sa zone centrale.

D'une façon avantageuse ladite aiguille est fixe et montée à proximité du dispositif régulateur de débit.

Ladite aiguille peut également être mobile et montée sur un support porte-aiguille mobile.

Dans une autre forme de réalisation le bouchon d'obturation amovible comporte une soupape unidirectionnelle.

Pour réduire au minimum le volume résiduel du réservoir, le bouchon d'obturation amovible et le piston mobile peuvent comporter respectivement au moins un évidement et une protubérance de forme et de dimensions complémentaires.

Dans toutes les variantes, le dispositif régulateur de débit est disposé à la base du distributeur, et de préférence dans l'embout applicateur de ce distributeur.

Dans une forme de réalisation avantageuse, le dispositif comporte au moins un évent disposé à l'extrémité du réservoir et débouchant dans la chambre de dérivation pour permettre l'évacuation de gaz contenus dans cette chambre avant l'utilisation de ce dispositif.

Le réservoir peut comporter deux compartiments séparés par un bouchon intermédiaire mobile, l'un des compartiments contenant un solvant et l'autre compartiment contenant une substance destinée à être dissoute ou mélangée au solvant.

Dans cette forme de réalisation, le réservoir comporte avantageusement un renflement central agencé pour permettre le passage du solvant liquide, lorsque le bouchon intermédiaire mobile est amené au voisinage de ce renflement.

Dans une forme de réalisation avantageuse, le dispositif comporte un capuchon de limitation de la course du poussoir pour assurer le mélange des composants.

Afin d' éliminer une évacuation résiduelle de liquide après l'utilisation, le piston peut comporter un plot de retenue rigide et une enveloppe souple définissant une cavité intérieure, ce plot de retenue comportant un téton d'appui et la hauteur totale dudit plot avec le téton d'appui étant sensiblement égale, au repos, à la hauteur de la cavité intérieure.

Dans la forme de réalisation préférée du dispositif le piston comporte un ressort spiral agencé pour appliquer une force de poussée progressive.

D'une façon avantageuse, dans toutes les formes de réalisation, le réservoir comporte une chemise cylindrique qui constitue ses parois latérales.

Selon une forme de réalisation particulièrement économique et efficace, le réservoir cylindrique se compose d'un premier élément tubulaire et d'un second élément tubulaire qui s'emboîtent l'un dans l'autre, le premier étant obturé par un obturateur fixe et par un piston mobile et le second contenant un élément rigide creux couplant mécaniquement le piston mobile et le piston coulissant, ledit obturateur fixe comportant une soupape unidirectionnelle permettant l'écoulement de la substance médicamenteuse vers le distributeur, et le fond dudit second élément tubulaire étant fermé.

Ledit réservoir cylindrique est avantageusement obturé par un piston coulissant solidaire d'un élément rigide creux monté au fond d'une capsule dans laquelle est emboîté partiellement ledit réservoir cylindrique, et ledit élément creux communique avec le distributeur à travers un filtre et/ou lesdits moyens d'aseptisation.

Selon un mode de réalisation avantageux, ledit organe régulateur de débit comporte un filtre de profondeur.

Ce filtre de profondeur peut être constitué d'un matériau fritté contenant au moins élément ayant un effet oligodynamique antibactérien, choisi parmi les métaux lourds, les composés ou les mélanges de ces métaux et les mélanges de ces composés.

Lesdits moyens d' aseptisation comportent de l'argent et/ou de l'oxyde d'argent.

Selon un mode de réalisation préféré, le dispositif est agencé pour que ledit volume prédéterminé soit inférieur au volume d'une goutte et de façon avantageuse, le rapport entre le volume prédéterminé mesuré en microlitres et la surface desdits moyens d'aseptisation mesurée en millimètres carrés est inférieur à 1.

Lesdits moyens élastiques comportent avantageusement une zone de moindre résistance ménagée du côté du piston en contact avec la substance médicamenteuse et agencée pour se déformer sous la poussée exercée sur ce piston et pour revenir dans son état normal à la fin de cette poussée.

La présente invention sera mieux comprise en référence à la description d'un exemple de réalisation et du dessin annexé dans lequel :
les figures 1A et 1B représentent une vue en coupe axiale d'une forme de réalisation du dispositif selon l'invention.
les figures 2 et 3 représentent une autre forme de réalisation du dispositif selon l'invention, respectivement en position de stockage et en position d'utilisation,
les figures 4 et 5 représentent une variante du dispositif selon l'invention, respectivement en position de stockage et en position d'utilisation,
les figures 6 et 7 représentent des vues de détail du dispositif selon l'invention,
les figures 8 et 9 représentent une autre variante du dispositif selon l'invention, respectivement en position de stockage et en position d'utilisation,
les figures 10 et 11 représentent également une autre variante du dispositif selon l'invention, respectivement en position de stockage et en position d'utilisation,
les figures 12 à 20 illustrent les différentes phases de préparation des éléments et d'assemblage de ces éléments pour aboutir aux dispositifs selon l'invention prêts au stockage,
la figure 21 représente une forme de réalisation particulière du dispositif selon l'invention, dont le réservoir comporte deux compartiments, ce dispositif étant représenté dans sa positon de stockage,
la figure 22 représente le dispositif de la figure 21 dans une position intermédiaire,
la figure 23 représente le dispositif selon l'invention en position d'utilisation,
les figures 24 à 33 illustrent les différentes phases de préparation des éléments et d'assemblage de ces éléments pour aboutir à un dispositif dont le réservoir contient deux composants, tels que représentés par les figures 21 à 23,
Les figures 34 à 36 illustrent une forme de réalisation préférée du piston respectivement au repos et au cours de l'utilisation,
les figures 37 et 38 représentent deux vues illustrant deux formes de réalisation du dispositif de l'invention dans son état de stockage,
la figure 39 représente une vue en coupe axiale d'une forme particulièrement avantageuse du dispositif de l'invention, en phase de stockage,
les figures 40 et 41 représentent la réalisation de la figure 39, respectivement en phase d'activation et en phase de distribution,
la figure 42 représente une variante de réalisation de celle illustrée par la figure 39, et
les figures 43 et 44 représentent des vues de détail illustrant le piston coulissant du dispositif selon la figure 42, respectivement au stockage et à l'utilisation.

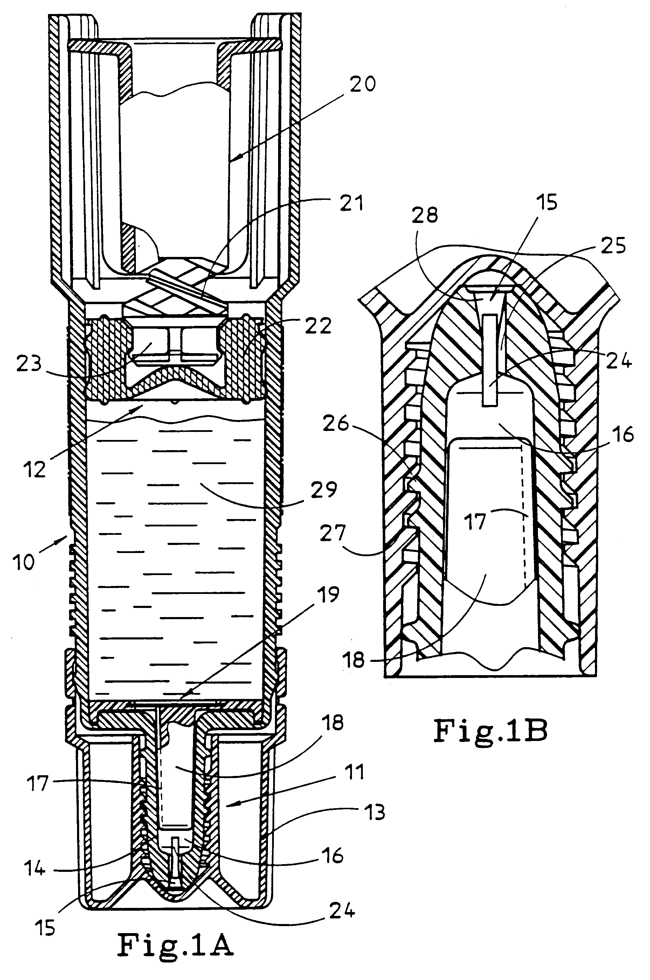

En référence aux figures 1A et 1B, le dispositif représenté sous sa forme comporte essentiellement un réservoir cylindrique 10, un distributeur 11 adapté à une extrémité de ce réservoir et un piston coulissant 12 engagé dans ce réservoir à son extrémité opposée au distributeur. Un capuchon protecteur 13 est monté par-dessus le distributeur 11.

Ce distributeur est constitué d'un embout applicateur 14 pourvu d'un orifice 15 qui est conçu pour permettre la formation de gouttes ophtalmiques destinées à être déposées dans l'oeil d'un patient. Cet orifice communique avec une chambre 16 ménagée à l'extrémité de l'embout applicateur, qui est elle-même en liaison avec l'intérieur du réservoir par un canal latéral 17 ménagé entre la paroi intérieure de l'embout applicateur et un insert 18, et à travers un dispositif régulateur de débit 19 qui sera décrit ci-dessous.

Ce dispositif régulateur de débit est constitué dans ce cas par une membrane ou filtre à micropores calibrés qui obture une extrémité du réservoir, l'autre extrémité étant obturée par le piston 12.

Ce piston est monté à l'extrémité d'une tige de piston 20 ou poussoir par l'intermédiaire d'un élément ressort 21 dont le rôle sera expliqué par la suite.

Le piston 12 se compose en fait d'un bouchon d'obturation 22 se présentant sous la forme d'une jupe annulaire réalisée en un matériau élastomère, qui est fixée à la périphérie d'un plot rigide 23 relié par l'intermédiaire de l'élément ressort 21 au poussoir 20.

Dans la forme de réalisation représentée, l'orifice 15 est constitué par l'extrémité d'un canal axial qui traverse la partie supérieure de l'embout applicateur. Dans ce canal est de préférence, logée une pointe d'argent 24 qui a des effets bactériostatiques par la libération de cations, et qui maintient ledit orifice de l'embout applicateur dans un état aseptique. Comme le montre plus particulièrement la figure 1B, cette tige d'argent 24 est maintenue dans une position axiale centrée au moyen d'au moins deux ailettes, radiales 25. Elle pourrait être remplacée par tout matériau équivalent ayant les mêmes fonctions ou même par un revêtement de même nature recouvrant la paroi intérieure de ce canal au voisinage de l'orifice 15.

Lorsque le capuchon protecteur 13 est mis en place sur le dispositif, c'est-à-dire lorsqu'il est vissé sur l'embout applicateur équipé d'un filetage mâle 26, le capuchon étant lui-même équipé d'un filetage femelle 27 correspondant, l'extrémité du canal qui constitue en fait la buse de l'embout applicateur définit au voisinage de l'orifice 15 une micro-chambre 28 dans laquelle peut subsister une partie de la solution médicamenteuse 29 contenue dans le réservoir 10, après une première utilisation. Cette quantité de substance de traitement est aseptisée grâce à la pointe d'argent avec laquelle elle est momentanément ou reste en contact.

Le dispositif régulateur de débit 19 est constitué par un ou plusieurs filtres à membrane comprenant des micropores calibrés, qui permet de contrôler de façon précise le débit de la substance médicamenteuse s'écoulant à travers le canal latéral en direction de l'orifice 15 de l'embout applicateur. Ce dispositif permet d'engendrer une perte de charge, à la traversée de la substance de traitement, qui est proportionnelle à la pression exercée sur la solution par le piston 12. Etant donné que la section du cylindre est relativement élevée, par exemple de l'ordre de 250 mm², il serait nécessaire d'appliquer une poussée de l'ordre de 2,5 kg pour atteindre une pression de l'ordre de 1 bar dans la solution. Toutefois, il est admis qu'en pratique une force de 1 kg est déjà considérée comme élevée pour activer le système, c'est-à-dire enfoncer le piston avec l'index. La pression maximale se situe approximativement à 0,4 bar et des tests démontrent que même en exerçant une force équivalente à 2 kg, il n'est pas possible de former un jet à l'extrémité de l'embout applicateur mais des gouttes unitaires conformément à ce qui était prévu. L'expérience a montré que grâce au dispositif régulateur de débit, on parvient à former des gouttes toutes les une à deux secondes au-de la de l'orifice 15. Ces gouttes ont un volume compris entre 20 et 40 µl.

L'élément ressort 21, qui ne peut pas être considéré comme indispensable, mais qui contribue au confort d'utilisation du dispositif, permet en fait de transmettre de façon progressive la poussée exercée par l'utilisateur sur la tige de piston ou poussoir. Il complète en quelque sorte la fonction du dispositif régulateur de débit en empêchant d'appliquer une poussée brutale sur la substance 29 contenue dans le réservoir 10.

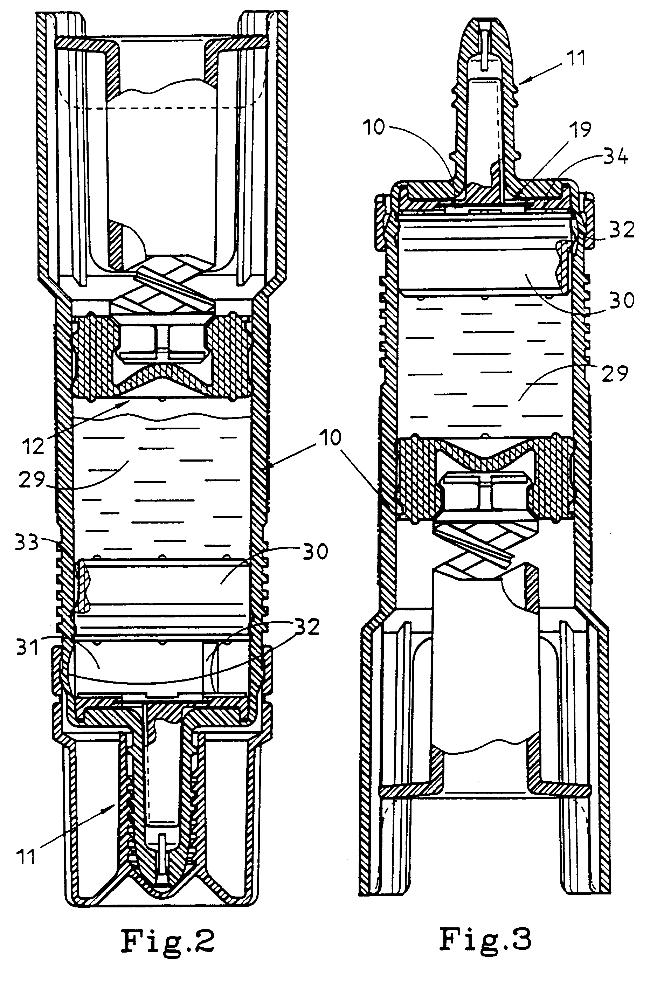

Les figures 2 et 3 illustrent une forme de réalisation dans laquelle le réservoir 10 est allongé et obturé, à proximité de son extrémité distale c'est-à-dire au voisinage du distributeur 11, par un bouchon d'obturation amovible 30, qui est destiné à être repoussé au moins partiellement dans une chambre de dérivation 31 au moment ou l'utilisateur enfonce le piston 12 en appuyant sur le poussoir 20.

La chambre de dérivation 31 comporte des évidements latéraux 32 qui permettent à la substance médicamenteuse contenue dans le réservoir 10 de s'écouler par un ou plusieurs canaux axiaux 33 ménagés entre le bouchon d'obturation amovible 30 et la paroi intérieure du réservoir, ces canaux communiquant avec les évidements 32 qui sont eux-mêmes en communication avec un ou plusieurs conduits radiaux 34 qui débouchent dans une zone centrale 35 localisée en amont du dispositif régulateur de débit 19 constitué par un filtre à membrane.

Cette réalisation est prévue pour le cas où la substance médicamenteuse contenue dans le réservoir ne supporte pas un contact de longue durée, qui peut aller jusqu'à trois ans, avec le dispositif régulateur de débit c'est-à-dire le filtre à membrane, alors que ce contact est tout à fait possible pendant la durée d'utilisation qui est de l'ordre d'un mois. Le bouchon d'obturation amovible a pour unique fonction de maintenir la substance médicamenteuse 29 dans un espace clos délimité par les parois du réservoir 10, la jupe annulaire du bouchon d'obturation 22 et ledit bouchon d'obturation amovible 30.

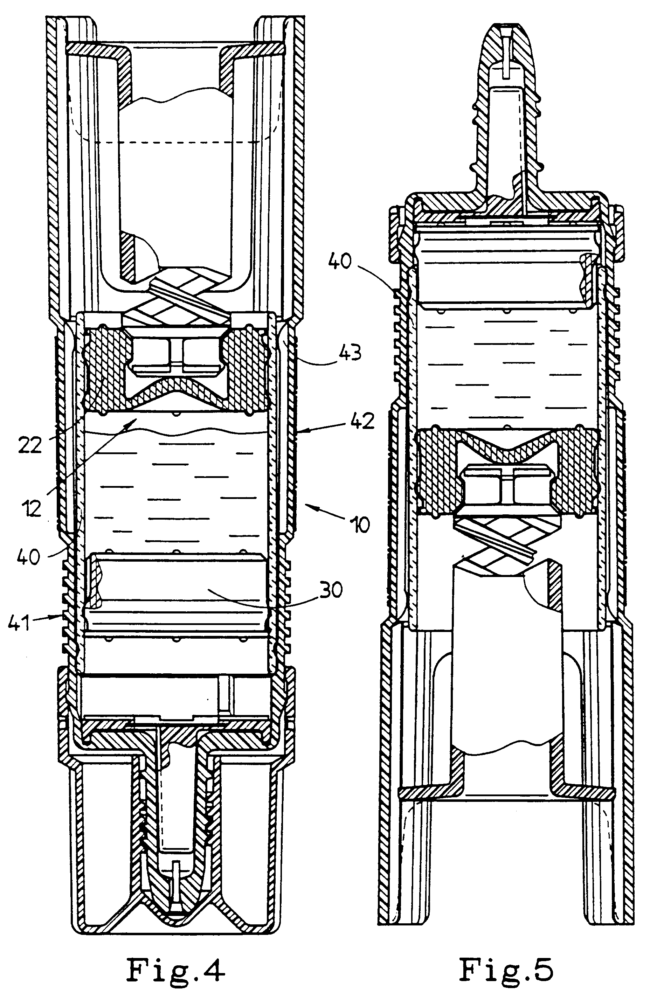

Les figures 4 et 5 illustrent une forme de réalisation qui diffère de la précédente en ce que le réservoir est chemisé ou, en d'autres termes, comporte une chemise cylindrique 40 en verre qui double les parois intérieures du réservoir. Dans cette réalisation, la substance médicamenteuse est emprisonnée dans un espace délimité par la chemise en verre 40, le bouchon d' obturation amovible 30 et le bouchon d'obturation 22 du piston 12.

Dans le but de faciliter la mise en place de ce réservoir autonome défini par les trois composants ci-dessus à l'intérieur du corps du réservoir 10, les parois de ce réservoir présentent avantageusement une première zone 41 dont la surface intérieure est lisse et a sensiblement le même diamètre que le diamètre extérieur de la chemise 40, et une deuxième zone 42 dont le diamètre intérieur est nettement supérieur à celui de la chemise 40. De ce fait la chemise 40 qui ne sera maintenue en place que par friction contre la paroi intérieure de la première zone 41, et éventuellement par des languettes de centrage 43 ménagées dans la deuxième zone 42, pourra être aisément mise en place, sans nécessiter une poussée exagérée.

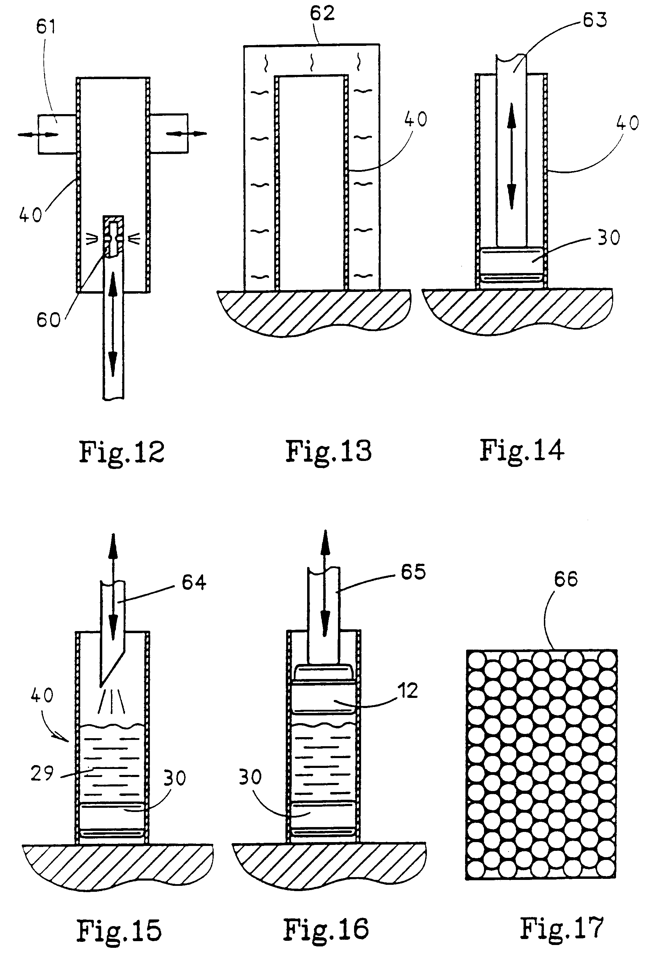

Dans cette réalisation il sera possible d'effectuer un remplissage entièrement automatisé du réservoir autonome, comme cela sera expliqué en référence aux figures 12 à 17, ce réservoir étant introduit dans le corps du dispositif en vue du stockage puis de l'utilisation de ce dernier.

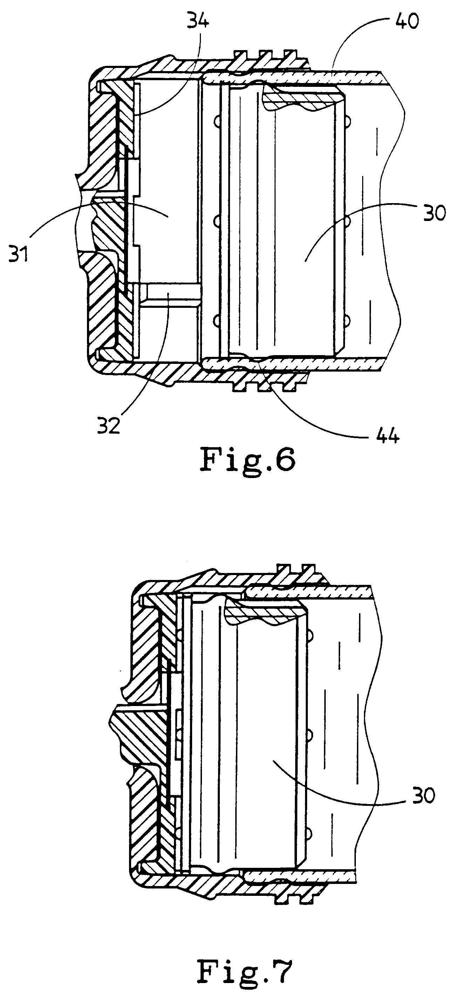

Les figures 6 et 7 sont des vues agrandies qui montrent de façon plus détaillée la forme de réalisation et le mode de fonctionnement du bouchon d'obturation amovible 30 dans le dispositif illustré par les figures 4 et 5. Ce bouchon d'obturation présente au moins un bourrelet annulaire 44 qui est partiellement écrasé lorsque ce bouchon d'obturation est dans sa position de stockage, c'est-à-dire engagé à l'intérieur de la chemise 40 du réservoir 10 et qui permet le passage de la substance médicamenteuse au cours de l'utilisation. Grâce aux évidements 32 similaires à ceux définis en référence à la figure 2, et à la position du bourrelet 44, la hauteur de la chambre de dérivation 31 est inférieure à la hauteur du bouchon d'obturation amovible, ce qui permet de réduire la longueur totale du corps du réservoir 10, par rapport à des réalisations dans lesquelles la chambre de dérivation doit avoir une hauteur suffisante pour loger entièrement ledit bouchon d'obturation amovible.

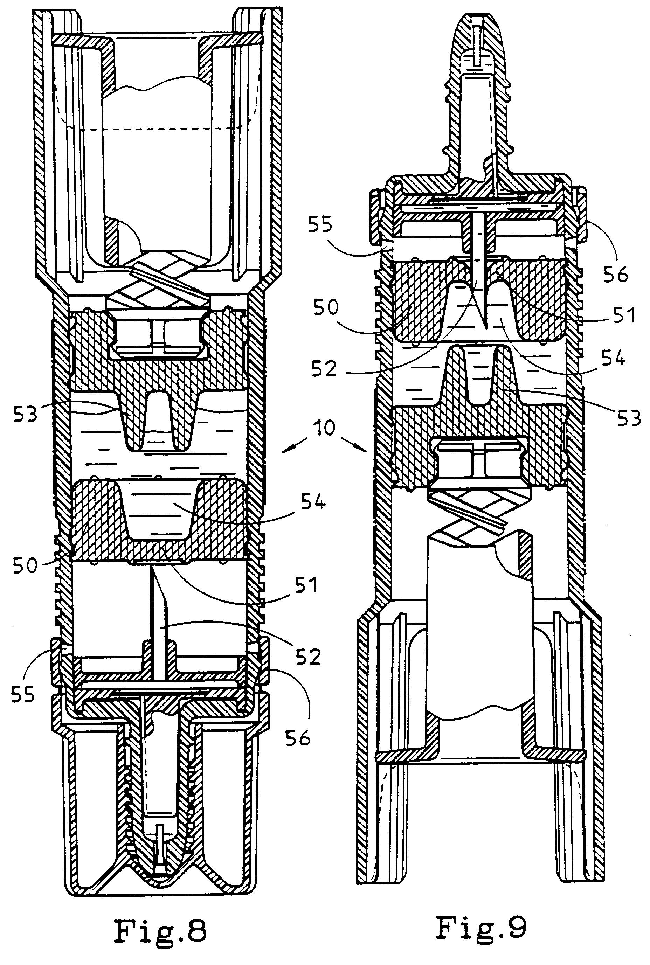

La construction illustrée par les figures 8 et 9 diffère de la réalisation précédente en ce que le bouchon d'obturation amovible est remplacé par un bouchon d' obturation 50 à percer. A cet effet, ce bouchon d'obturation comporte une zone centrale d'épaisseur réduite 51 et une aiguille 52 est montée au fond du réservoir en regard de l'embout applicateur. La jupe annulaire du piston présente deux protubérances 53 ayant une forme sensiblement complémentaire à celle de l'évidement 54 du bouchon d'obturation amovible dans la zone centrale de moindre épaisseur 51.

Des orifices latéraux 55 servant d'évents, obturés pendant la phase de stockage par le capuchon protecteur 15, permettent l'évacuation des gaz initialement contenus dans le réservoir en aval du bouchon d'obturation amovible 50 lorsque ce bouchon est déplacé et lorsque le capuchon est dévissé. En fait ces ouvertures 55 sont obturées par une partie inférieure du capuchon protecteur, cette partie inférieure constituant une bague d'inviolabilité 56 rattachée au reste du capuchon par des bras de liaison qui se brisent pour dégager ces orifices lorsque l'utilisateur veut procéder à l'activation du dispositif.

Il serait également envisageable de modifier cette construction en montant l'aiguille 52 sur un support porte-aiguille mobile qui pourrait être enfoncé vers l'intérieur du réservoir pour assurer la perforation du bouchon d'obturation.

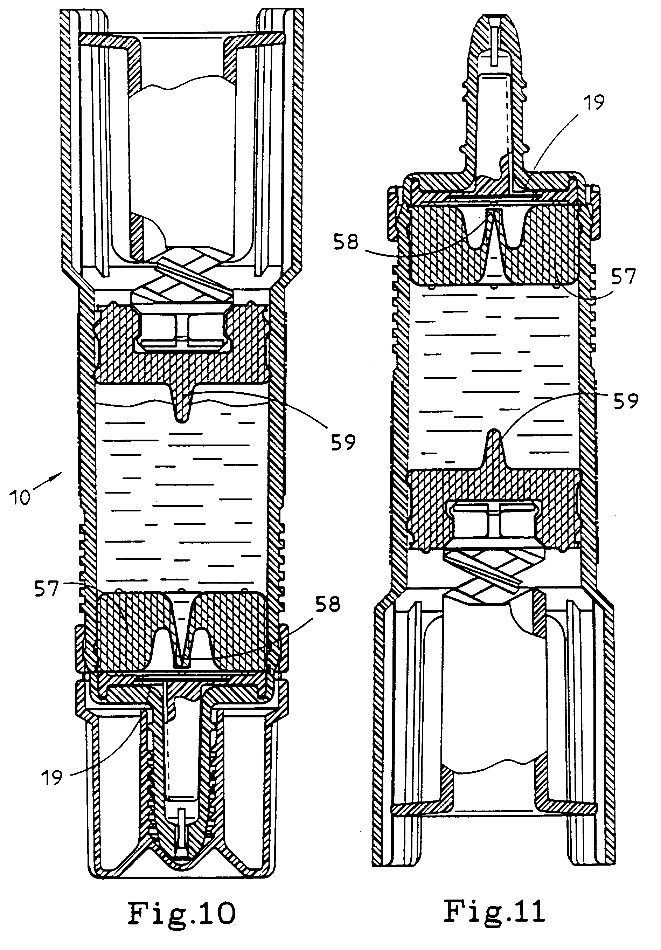

Les figures 10 et 11 illustrent une autre forme de réalisation, dérivée de celle des figures 8 et 9, dans laquelle le bouchon d'obturation amovible 57 est pourvu, dans sa zone centrale, d'une soupape unidirectionnelle 58 constituée par une fente étroite qui, en l'absence de pression exercée sur la substance médicamenteuse 29, est fermée et ne s'ouvre pour laisser passer cette substance vers le dispositif régulateur de débit 19, que lorsque la pression est suffisante pour ouvrir les lèvres de cette soupape unidirectionnelle.

La jupe annulaire du piston présente dans ce cas une protubérance 59 dont la géométrie est de préférence complémentaire à celle de l'évidement ménagé entre les lèvres de la soupape unidirectionnelle de telle façon, qu'en fin d' utilisation, cette protubérance pénètre dans cet évidement pour réduire au minimum le volume mort ou volume résiduel du réservoir.

Dans toutes les réalisations précédentes, le dispositif régulateur de débit a été représenté sous la forme d'un filtre à membrane comportant un nombre prédéterminé de micropores calibrés. Il est à noter que ce type de dispositif régulateur de débit pourrait être remplacé par tout autre moyen approprié tel que par exemple un orifice calibré suffisamment petit, un canal étranglé rectiligne ou en spirale ou tout autre moyen approprié permettant de modérer le débit afin d'éviter la formation d'un jet au profit de gouttes régulières, et ceci quelle que soit la pression exercée sur le piston.

Le filtre à membrane est avantageusement un filtre stérilisant dont les pores peuvent avoir un diamètre compris entre 0,1 et 0,45 microns et de préférence voisin de 0,22 microns de manière à arrêter toutes les particules solides, ainsi que les bactéries ou autres corps étrangers dont les dimensions sont supérieures à ces valeurs.

Dans toutes ces réalisations, il est prévu au moins un filtre qui a principalement une fonction de régulateur de débit, et parallèlement une fonction de filtre anti-particulaire, antiseptique et absorbant les agents conservateurs. Le dispositif peut éventuellement comporter un deuxième organe tel que par exemple un filtre à membrane ou un tampon pour absorber les agents conservateurs.

Les avantages à l'utilisation découlent de manière directe des constructions décrites ci-dessus. Parmi les avantages directement perceptibles par l'utilisateur, celui de la régularité de la précision des gouttes, que l'on forme à l'extrémité de l'embout applicateur lorsque l'on appuie sur le poussoir, n'est pas le moindre. En effet, il est essentiel que les gouttes aient un volume prédéterminé qui reste constant tout au long de l'utilisation du dispositif. Le filtre régulateur de débit qui sert de frein et empêche l'évacuation de la substance médicamenteuse sous forme de jet assure cette régularité. Cette régularité est aussi obtenue grâce au poussoir en forme de ressort qui constitue un moyen pour démultiplier sa course par rapport à celle du piston. En pratique, lorsque l'utilisateur appuie sur le poussoir, du fait de l'élasticité du ressort, il peut le comprimer d'une certaine longueur avant de provoquer un déplacement du piston. Le filtre impose une compression limite à partir de laquelle la substance le traverse pour former une goutte calibrée. Par ailleurs ce filtre a également une fonction anti-particulaire qui empêche le passage de toutes particules solides provenant soit du mélange de médicament ayant servi à la constitution de la substance médicamenteuse de traitement, soit des composants du dispositif. Parmi les particules susceptibles de provenir de la substance médicamenteuse elle-même, on peut citer les particules mal dissoutes ou mal mélangées lors de la fabrication du produit ou les particules dues à des polymérisations qui peuvent se produire pendant le stockage du dispositif.

Enfin, certaines substances ne supportent pas la stérilisation à haute température, de sorte que leur conservation implique l'introduction d'au moins un agent conservateur pendant le stockage à moins que l'on puisse se satisfaire d'un remplissage aseptique. Un filtre, ou un tampon spécialement conçu pour absorber cet agent conservateur avant l'utilisation de la substance, peut être logé dans l'embout applicateur. En effet ces agents conservateurs, qui peuvent créer des allergies, provoquer des irritations ou des effets secondaires, doivent impérativement être éliminés avant l'introduction de la substance médicamenteuse dans l'oeil du patient.

Le filtre placé à l'extrémité de l'embout applicateur a pour effet d'éviter une contamination due à la migration bactérienne provenant de l'extérieur et se propageant vers l'intérieur de l'embout applicateur. Enfin, le cache muni d'une tige d'argent ou de tout autre matériau approprié permet d'assurer en permanence une désinfection active de l'extrémité distale de l'embout applicateur.

Les avantages pour le fabricant du dispositif représenté par la réalisation illustrée en partie par les figures 4 à 7 dans laquelle le réservoir est chemisé apparaîtront plus en détail en référence aux figures 12 à 20. Ces figures illustrent tout le processus de fabrication du dispositif, c'est-à-dire de la préparation et de l'assemblage des différents composants.

La figure 12 représente la phase qui consiste à nettoyer et à enduire de silicone au moyen de buses rétractables 60 la chemise cylindrique 40 tenue, pendant cette opération, par une pince 61. Cette chemise cylindrique 40 est découpée au préalable par choc thermique dans un tube en verre et ne subit aucune transformation particulière. Cette chemise cylindrique subit ensuite une recuisson et une dépyrogénation à 300°C dans un four 62 schématiquement représenté par la figure 13. La figure 14 illustre la mise en place du bouchon d'obturation amovible 30 au moyen d'un poussoir 63 mobile axialement. La figure 15 représente la phase de remplissage du réservoir ou plus exactement de la chemise 40 par la substance médicamenteuse 29 amenée par un injecteur rétractable 64. La figure 16 représente la mise en place du piston 12 au moyen d'un poussoir 65 mobile axialement. Etant donné que le réservoir a une forme extérieure parfaitement cylindrique, une quantité importante de ces réservoirs préalablement remplis peut être juxtaposée et placée dans un magasin 66 en vue de traitements ultérieurs. Parmi ces traitements ultérieurs l'un des plus appréciés est l'autoclavage qui est pratiqué à condition que la substance médicamenteuse 29 supporte l'élévation de température correspondante.

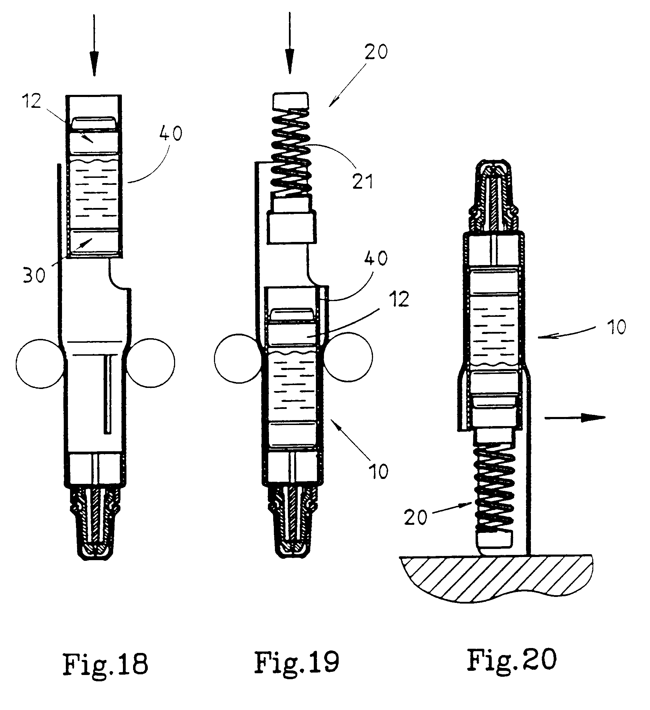

La figure 18 illustre la mise en place du réservoir 10 dans le dispositif qui a été préalablement stérilisé de façon appropriée. Après cette mise en place, le poussoir 20 est amené en position et couplé au piston comme le montre la figure 19. La figure 20 montre le dispositif entièrement assemblé et retourné. Dans cette positon, il peut également être stocké en magasin en vue d'un conditionnement individuel ou collectif.

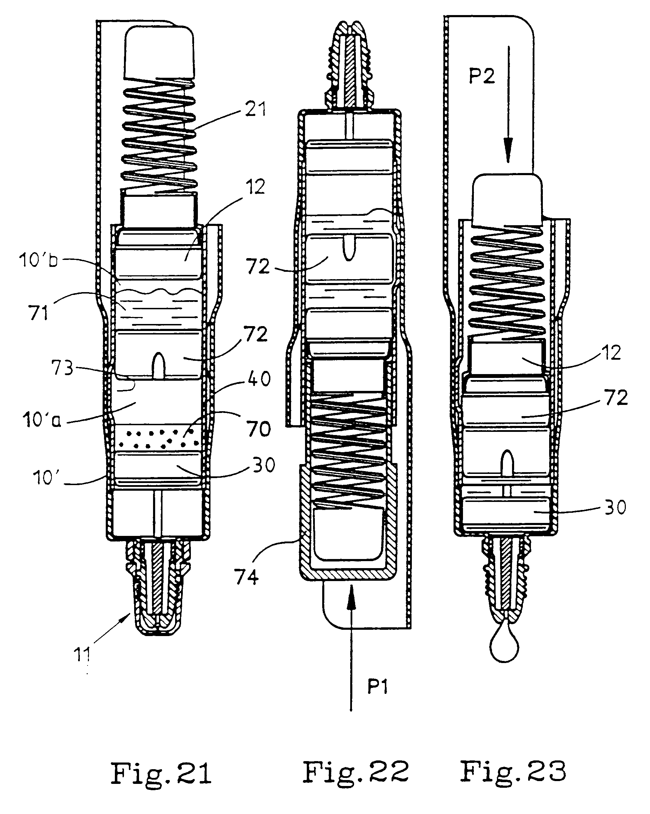

Les figures 21 à 23 représentent un dispositif de stockage et de distribution de gouttes ophtalmiques dans lequel le réservoir 10' est subdivisé en deux compartiments respectivement 10'a et 10'b, dont le compartiment inférieur 10'a peut contenir soit un lyophilisat 70 (comme le montre la figure), soit une poudre, soit un liquide et le compartiment supérieur 10'b contient obligatoirement un solvant 71 à l'état liquide. Comme précédemment le réservoir 10' est obturé à une de ses extrémités par un bouchon d' obturation amovible 30 et à son extrémité supérieure par un piston 12. Un bouchon intermédiaire mobile 72 sépare les deux compartiments 10'a et 10'b. Dans cette forme de réalisation, la chemise cylindrique 40 du réservoir 10' présente dans sa partie centrale un renflement 73 qui constitue un by-pass au moment de l'activation du système, qui sera décrite ci-dessous.

Les autres éléments tels que le poussoir 20 et l'embout applicateur 14 sont inchangés par rapport à ceux décrits ci-dessus.

L'activation du dispositif est illustrée par la figure 22. Une première poussée P₁ sur le poussoir 20 provoque le déplacement du bouchon intermédiaire mobile 72 dans la zone centrale du réservoir dans laquelle la chemise cylindrique comporte le renflement 73. De ce fait, le solvant 71 passe dans le compartiment 10'a pour dissoudre la poudre ou le lyophilisat 70. Par ce moyen, on constitue ou on reconstitue la substance médicamenteuse qui sera par la suite utilisée pour former les gouttes à déposer dans l'oeil d'un patient. Le poussoir 20 peut être équipé d'une butée amovible sous la forme d'un capuchon 74 limitant la course nécessaire au mélange des composants.

La phase suivante, qui est la phase d'utilisation, est illustrée par la figure 23. A la fin de la phase précédente, lorsque tout le solvant est passé dans le compartiment 10'a, le piston 20 est en butée contre l'obturateur intermédiaire mobile 72 qui ne constitue plus qu'un seul élément ayant la fonction d'un piston. Après avoir retiré la butée amovible, le fonctionnement à l'utilisation du dispositif est strictement identique à celui qui est prévu pour une substance à un seul composant qui a été décrite précédemment. Une poussée P₂ sur le piston, après évacuation préliminaire des gaz contenus dans la chambre de dérivation a pour effet de repousser le bouchon d'obturation amovible 30 dans la chambre de dérivation 31 pour autant que ledit bouchon d'obturation amovible ne se soit pas déjà déplacé dans ladite chambre de dérivation, puis de permettre le passage de la substance médicamenteuse précédemment reconstituée pour former les gouttes ophtalmiques.

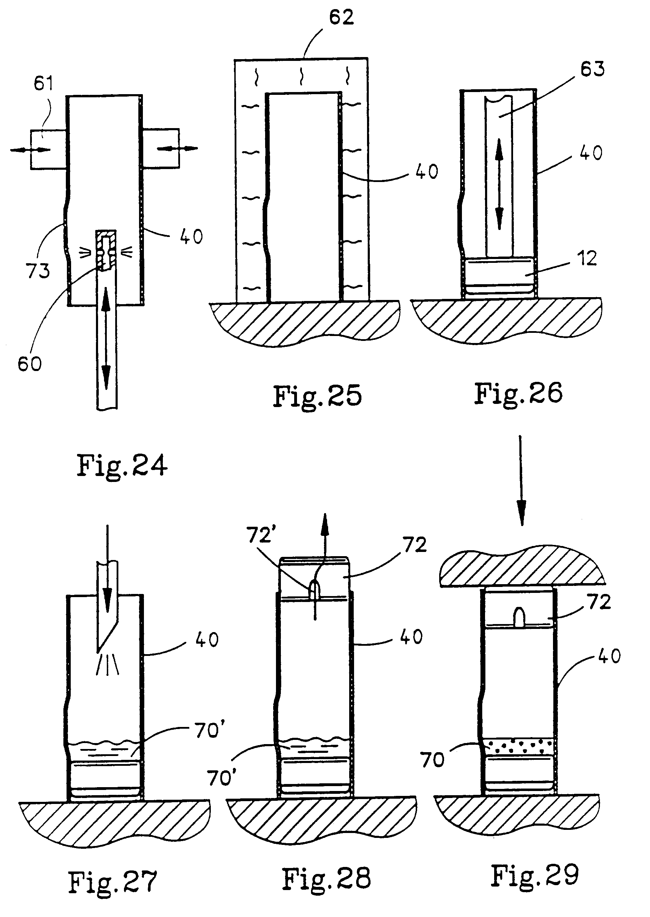

Les figures 24 à 33 illustrent les différentes phases de préparation et d'assemblage des composants du dispositif décrit précédemment dans lequel le réservoir est du type à mélange de deux composants. La première phase illustrée par la figure 24 représente comme précédemment le lavage et l'enduction d'un film de silicone de la chemise 40 préalablement découpée dans un tube cylindrique en verre, dans lequel on a formé le renflement 73 par un procédé de chauffage local permettant d'effectuer la déformation appropriée. La phase suivante représentée par la figure 25 consiste à recuire et à dépyrogéner cette chemise dans un four 62 à environ 300°C. La phase suivante, représentée par la figure 26, est identique à celle décrite en référence à la figure 14. La figure 27 consiste à mettre en place un premier composant qui sera ensuite soumis à une phase de lyophilisation. Ce premier composant 70' aboutira, après la phase de lyophilisation représentée par la figure 28, au lyophilisat 70 contenu dans le compartiment 10'a. On notera qu'au cours de la phase de lyophilisation, le bouchon intermédiaire mobile 72 est prépositionné sur la chemise 17. Pour permettre l'évacuation des gaz, l'obturateur intermédiaire mobile 72 comporte au moins un évent latéral 72' adapté à cet effet. Au cours de la phase suivante représentée par la figure 29, cet obturateur intermédiaire mobile 72 est engagé dans la chemise cylindrique 40 d'une manière suffisante pour l'obturer.

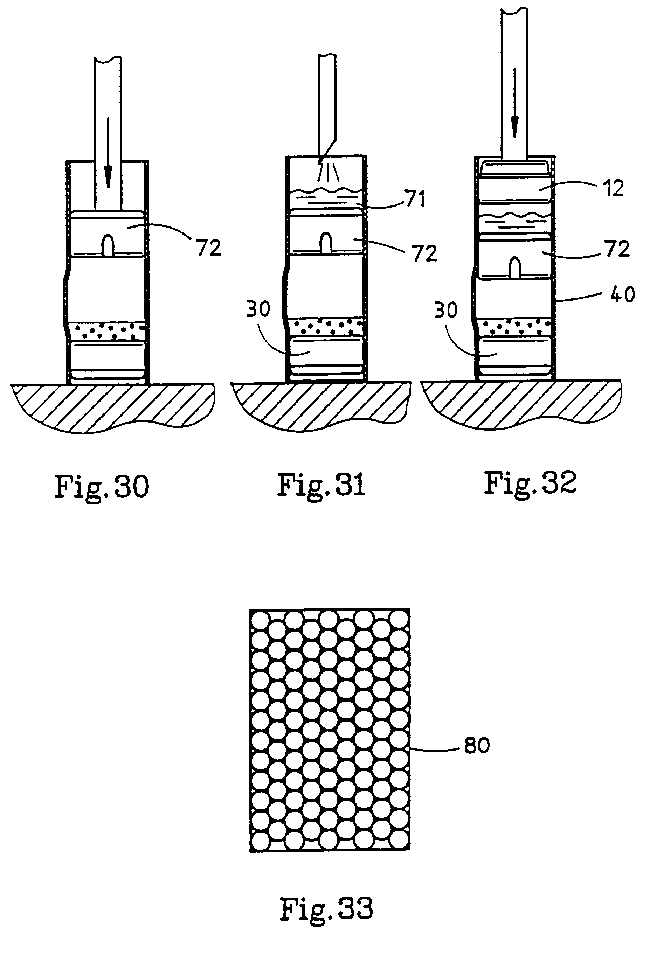

Au cours de la phase représentée par la figure 30, un poussoir repousse l'obturateur intermédiaire mobile 72 dans sa position centrale qu'il occupera pendant toute la phase de stockage du dispositif. Après la mise en place de cet organe, le solvant 71 est versé dans le compartiment supérieur délimité vers le bas, par l'obturateur intermédiaire mobile. Au cours de la phase suivante représentée par la figure 32, le piston 20 est mis en place pour assurer l'obturation finale de la chemise cylindrique 40. Dans cet état, les réservoirs remplis ainsi formés peuvent être disposés en cassettes ou magasins 80 (voir figure 33) en vue de divers traitements ou diverses manipulations ultérieures. Il est bien entendu que dans ce cas une stérilisation des réservoirs n'est pas possible en raison de l'existence du lyophilisat qui ne supporte pas une telle opération.

La mise en place du réservoir dans le corps du dispositif et le montage du poussoir lorsque ce réservoir est en place pour rendre ce dispositif prêt au stockage, constituent des opérations identiques à celles décrites ci-dessus en référence aux figures 18 à 20.

Il a été mentionné précédemment que le lyophilisat pouvait être remplacé soit par une poudre, soit par un liquide. La plupart des phases opératoires destinées à mettre en place les substances dans les deux compartiments sont identiques à celles décrites en référence aux figures 24 à 33. Toutefois, lorsque l'un des compartiments contient une poudre, il est nécessaire de mettre d'abord en place le solvant dans le compartiment supérieur, puis de positionner l'obturateur intermédiaire mobile et enfin de mettre la poudre en place dans le compartiment inférieur. Cette inversion des opérations par rapport à celles qui consistent à mettre en place le lyophilisat et le solvant est rendue nécessaire pour éviter une contamination du compartiment supérieur par la poudre. Une phase de décontamination du réservoir est d'ailleurs requise pour éliminer les particules de poudre qui auraient pu se fixer à l'extérieur de ce réservoir. On notera que dans ce cas, une stérilisation n'est généralement pas possible parce que la poudre n'est pas en mesure de supporter une telle opération.

En revanche, lorsque les deux substances contenues respectivement dans le compartiment inférieur et le compartiment supérieur sont des solutions à l'état liquide, le remplissage peut se faire indifféremment dans un sens ou dans l'autre, c'est-à-dire commencer soit par le compartiment supérieur, soit par le compartiment inférieur. En outre, pour autant que les solutions supportent une élévation de température sans transformation chimique ou décomposition, une stérilisation complète du réservoir est possible.

L'utilisation dans les deux cas est identique à celle décrite précédemment pour le cas où l'un des compartiment contient un lyophilisat.

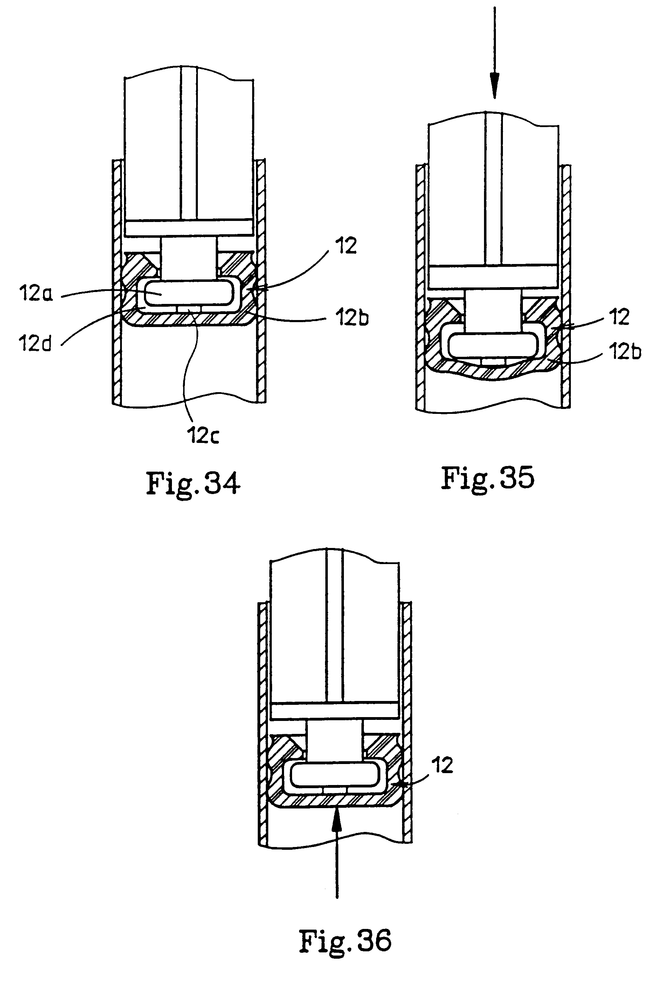

Pour toutes ces réalisations, il peut subsister un problème qui est le suivant. Lorsque l'utilisateur appuie sur le piston, la compression d'un faible volume d'air contenu dans le réservoir peut, après le dépôt d'une goutte ophtalmique, repousser une certaine quantité de liquide hors de l'embout applicateur, avec tous les inconvénients que cela peut comporter. Pour éviter ce défaut on crée une mini aspiration en fin d'utilisation pour réaspirer le volume de liquide qui aurait tendance à s'échapper de manière incontrôlée. Cet effet est obtenu grâce à une construction particulière du piston 12. Comme le montre la figure 34, le piston 12 comporte un plot de retenue rigide 12a qui est solidaire de la tige de piston et une jupe annulaire souple 12b qui entoure ce plot. Un téton d'appui 12c est disposé à la base du plot de retenue 12a de telle manière qu'au repos, la hauteur totale du plot y compris le téton 12c correspond approximativement à celle de la cavité intérieure 12d de la jupe annulaire 12b. Lorsque l'on appuie sur le piston, le téton 12c provoque une déformation à la base de la jupe annulaire comme le montre la figure 35. A la fin de l'utilisation, lorsque la jupe reprend sa position initiale, la dépression créée dans le réservoir engendre une mini-aspiration, comme le montre la figure 36. Le but recherché est ainsi atteint.

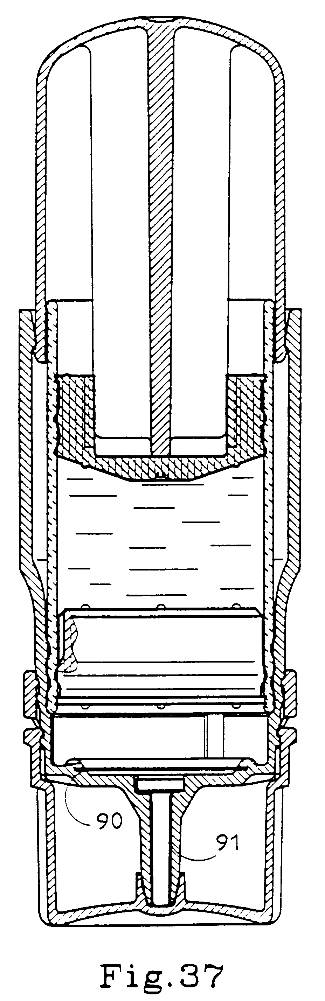

En référence à la figure 37, le dispositif comporte un filtre 90 monté à la base de l'embout applicateur. Ce filtre a une surface relativement grande. A l'intérieur de l'embout applicateur est monté un insert 91 fritté en oxyde d'argent qui a une fonction stérilisante.

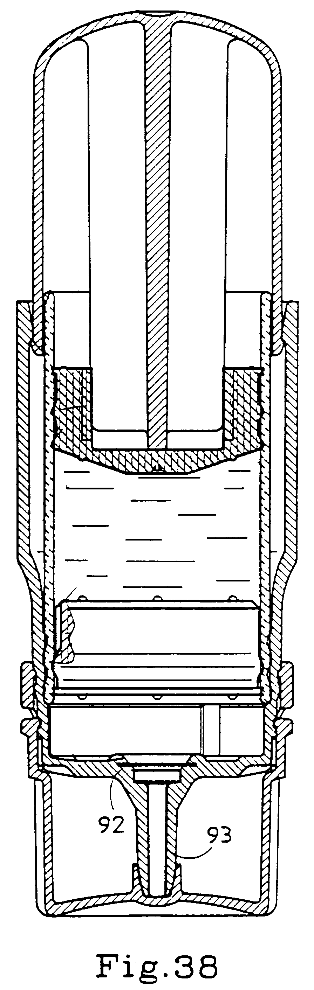

Dans l'exemple représenté par la figure 38, le filtre 92, qui a avantageusement des fonctions stérilisantes, a une surface plus faible. Un insert en argent 93 est avantageusement placé à l'intérieur de l'embout applicateur. Dans les deux cas l'insert définit un canal axial permettant l'écoulement de la substance de traitement.

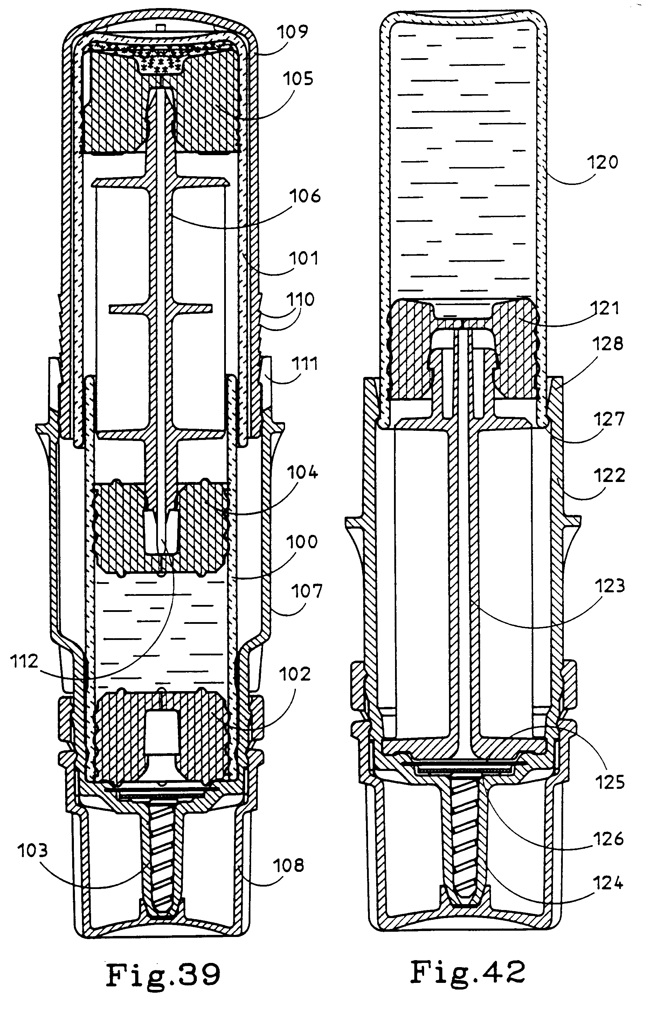

La figure 39 illustre une autre forme de réalisation qui comprend un premier élément tubulaire 100 qui est partiellement emboîté dans un second élément tubulaire 101 à fond fermé. Le premier élément tubulaire est obturé par un obturateur fixe 102 disposé à une de ses extrémités voisine d'un embout applicateur 103 du dispositif et par un piston mobile 104. L'espace intérieur à ce premier élément tubulaire et délimité par l'obturateur fixe 102 et le piston mobile 104 contient la substance médicamenteuse. Le second élément tubulaire contient un piston coulissant 105 qui est mécaniquement couplé au piston mobile 104 par un élément rigide 106 creux définissant un canal d'écoulement de la substance médicamenteuse.

Ledit premier élément tubulaire 100 est logé dans une capsule 107 qui se termine par l'embout applicateur 103 coiffé par un capuchon protecteur amovible 108. Ledit second élément tubulaire 101 est coiffé par un capuchon 109 à fond fermé qui est emboîté dans la capsule 107. Ce capuchon 109 comporte des crans 110 ou des rainures périphériques et la capsule comporte deux ou plusieurs languettes élastiques 111 pourvues d'un bec destiné à coopérer avec les crans 110 pour empêcher le déplacement relatif du capuchon 109 hors de la capsule tout en autorisant le déplacement en sens contraire.

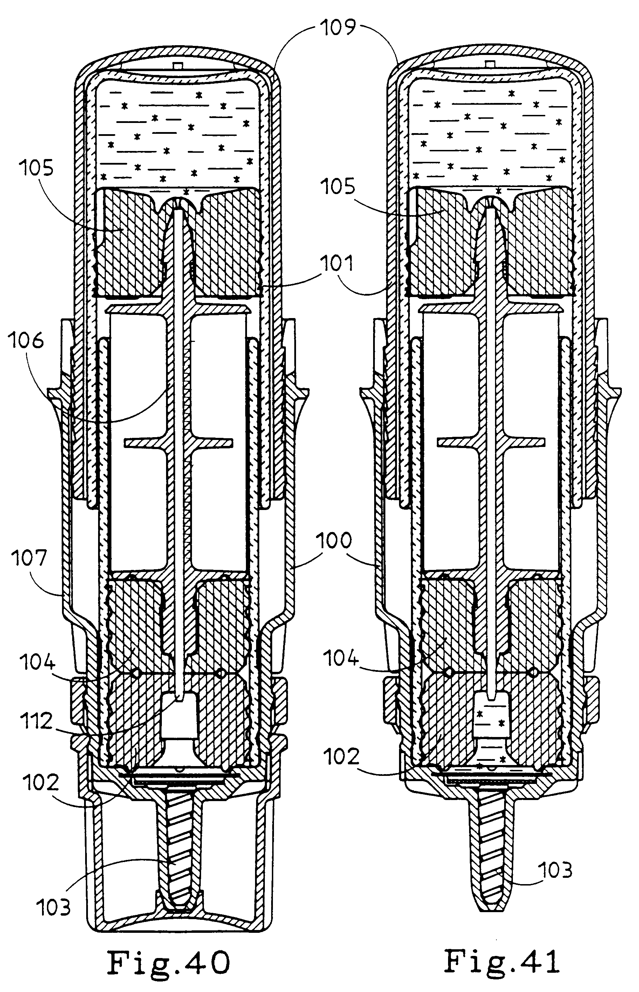

Comme le montrent les figures 40 et 41, pour activer le dispositif il convient de pousser le second élément tubulaire en direction du premier en appuyant sur le fond du capuchon 109. Ceci a pour effet de faire avancer l'élément rigide 106 dont la pointe 112 traverse le piston mobile 104 dans sa zone centrale prépercée, et de refouler la substance médicamenteuse dans une chambre qui se dégage à l'arrière du second élément tubulaire 101 grâce au déplacement du piston coulissant 105. De ce fait, l'ensemble de la substance liquide se transfère de l'avant à l'arrière du dispositif. Le piston mobile se déplace vers l'avant et se bloque contre l'obturateur 102. Le dispositif est prêt à l'utilisation. Une poussée supplémentaire sur le fond du dispositif provoque l'écoulement goutte à goutte de la substance médicamenteuse.

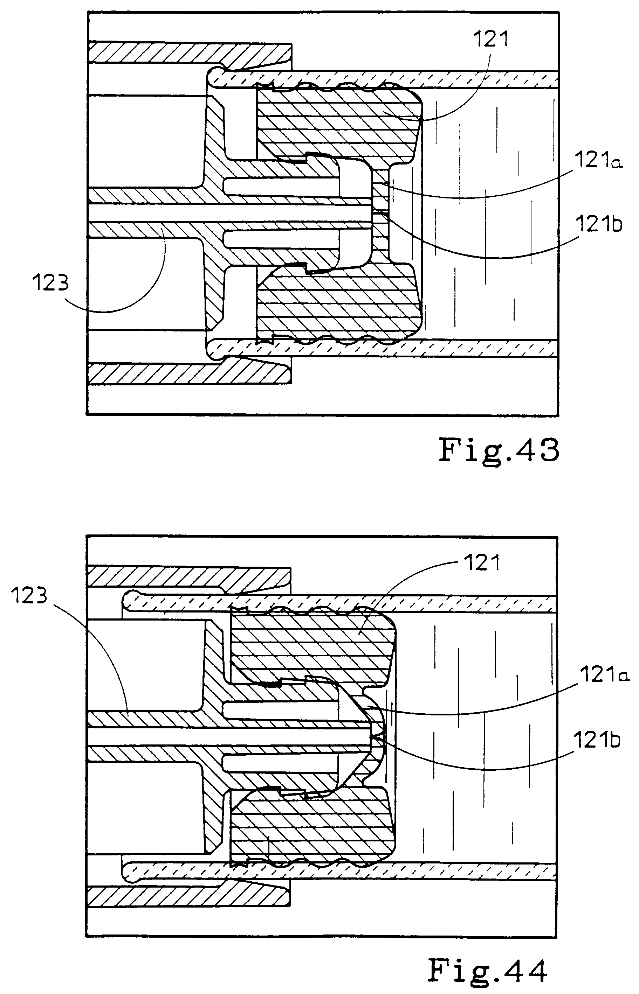

La figure 42 représente une variante du dispositif selon les figures 39 à 41. Dans cette réalisation un conteneur 120 dans lequel est stockée la substance médicamenteuse est obturé par un piston coulissant 121 et est engagé dans une capsule 122 sensiblement similaire à la capsule 107 du dispositif des figures précédentes. Le piston coulissant 121 est couplé mécaniquement avec la capsule 122 par un élément rigide 123 fixé au fond de cette capsule et définissant avec cette dernière un évidement central communiquant avec un embout applicateur 124. Dans cet évidement sont fixés un filtre 125 et une pastille d'argent 126. Le conteneur comporte un bourrelet périphérique 127 coopérant avec des languettes de retenue 128 solidaires de la capsule 122 pour retenir le conteneur dans la capsule.

Le détail de la forme du piston coulissant 121 est illustré par les figures 43 et 44, respectivement au stockage et à l'utilisation. On constate que la partie centrale 121a du piston coulissant 121 est une membrane qui se déforme sous la poussée de l'extrémité de l'élément rigide 123 pendant la phase d'utilisation (fig. 44), ce qui a pour effet d'ouvrir la soupape unidirectionnelle 121b et de permettre l'écoulement de la substance médicamenteuse.

On constate que dans toutes les formes de réalisation décrites et illustrées par les figures, on retrouve en totalité ou partiellement un certains nombre de moyens correspondant aux objectifs du dispositif idéal définit précédemment par le cahier des charges. Parmi ces moyens on compte des moyens permettant de générer des gouttes ophtalmiques avec une force constante. Ces moyens comportent un piston mobile se déplaçant axialement dans le sens permettant de provoquer une diminution du volume dans un conteneur dans lequel est stockée la substance médicamenteuse ou un piston fixe et un conteneur mobile coulissant par-dessus le piston fixe et assurant une diminution du volume de la chambre contenant la substance médicamenteuse stockée dans ce récipient mobile.

On compte également des moyens permettant de proscrire la possibilité de générer une dépression à l'intérieur du récipient. Ces moyens comportent un piston mobile et une tige de piston non couplés mécaniquement ou un piston mobile à soupape unidirectionnelle qui s'obture lors du retrait du récipient mobile, ou un obturateur fixe à soupape unidirectionnelle ou un poussoir mobile recouvrant le récipient mobile sans liaison avec celui-ci et empêchant toute possibilité de retirer le récipient mobile en agissant par traction sur ce dernier, de telle manière que seul un déplacement dans une seule direction par poussée sur le récipient soit possible.

On compte également des moyens permettant de maîtriser le débit sans risque de formation de jet pour générer des gouttes régulières et précises. Ces moyens comportent un régulateur de débit créant une perte de charge proportionnelle à la pression et à la viscosité de la solution sous la forme d'un filtre de profondeur synthétique poreux, obtenu par exemple par compression de poudre ou de billes de métal, puis fritté dans lequel sont perforés des trous calibrés ou des passages à chicanes etc.. Ce moyen est considéré comme indispensable pour obtenir le résultat escompté parce qu'il est impossible de maîtriser de manière précise le déplacement du piston, déplacement qui est de l'ordre du dixième de millimètre pour générer une goutte de 30 µl, en raison des forces de friction du piston contre les parois du récipient. Des que cette force de friction est vaincue le coulissement est inévitable et la solution pourrait s'écouler librement sous la forme de jet en l'absence d'un régulateur de débit.

On notera que ce régulateur de débit dans un dispositif à piston a le même effet que celui obtenu lorsqu'on exerce une pression sur les parois d'un conteneur souple tel qu'il est couramment utilisé dans les dispositifs connus.

Il compte également des moyens permettant de supprimer la pression résiduelle à l'intérieur du récipient après l'administration précise d'une goutte due au déplacement du piston, en générant une pression résiduelle dans le réservoir, pression résultant de la force de friction du piston contre les parois du réservoir et de la perte de charge induite par le régulateur de débit. Ces moyens ont pour but d'éviter la formation d'une deuxième goutte a l'extrémité de l'embout applicateur au moment de la première goutte. Ces moyens comportent une zone centrale ayant l'aspect d'une membrane élastique ménagée au centre du piston coulissant.

Il compte également des moyens permettant d' obturer l'embout à son extrémité de manière étanche, avec garantie d'étanchéité et sans générer de surpression ni de refoulement de la colonne de liquide se trouvant dans l'embout applicateur vers l'intérieur du récipient. Ces moyens comportent un capuchon non vissé. Dans les systèmes connus, le capuchon vissé est généralement pourvu d'une bague d'étanchéité obturant le flacon avant le vissage complet du capuchon pour éviter un défaut d' étanchéité dû au vissage imparfait de ce capuchon. Cette construction présente l'inconvénient qu'elle génère une surpression et engendre un refoulement de la colonne liquide vers l'intérieur du conteneur ce qui est à l'origine de contamination bactérienne ou de formation de mousse due à la pénétration d'air dans le conteneur.

Il compte en outre des moyens pour supprimer les turbulences dans l'embout applicateur. Parmi ces moyens on peut citer une soupape unidirectionnelle et un piston à déplacement unidirectionnel empêchant une réaspiration du liquide vers l'intérieur du conteneur.

Il compte encore des moyens permettant de proscrire la migration de germes vers l'intérieur de l'embout applicateur et vers l'intérieur du conteneur. Parmi ces moyens on note la présence d'un filtre de profondeur fritté de chicanes, d' ouvertures capillaires, d'un filtre stérilisant à membrane, d'un filtre en céramique poreux ou d'un filtre en verre fritté.

Il compte enfin des moyens permettant de supprimer la prolifération des germes ayant éventuellement migré à l'intérieur de l'embout après la distribution d'une goutte. Ces moyens sont obtenus par la présence de matériaux ayant une action autostérilisante bactéricide ou bactériostatique visant à inhiber la prolifération des germes par un effet oligodynamique. Ces moyens comportent un élément, un revêtement ou un insert poreux ou non réalisé en métaux lourds, par exemple en métaux précieux ou semi-précieux tels que par exemple l'argent, l'or, le platine, l'étain, le cuivre etc., ou en composés de ces métaux, notamment en oxydes ou mélanges d'oxydes tels que l'oxyde d'argent, ou en d'autres matériaux tels que la céramique, le Téflon ® ou toute autre substance ayant des effets similaires amorphes ou hydrophobes, ou enfin en mélange ou combinaison des métaux, composés ou substances précités. Parmi ces moyens, on compte également une construction particulière de l' embout applicateur. Cette construction particulière permet de ne maintenir qu'un volume résiduel extrêmement faible après la formation et l'expulsion d'une goutte. A ce propos, on notera que le volume résiduel est idéalement de l'ordre de 10 à 20% du volume d'une goutte, de sorte que la perte reste minime et la précision de dosage suffisamment grande même lorsque l'effet oligodynamique dénature en partie les composants actifs du volume résiduel mentionné ci-dessus.

Une solution préférée consiste à insérer une tige poreuse à l'intérieur de l'embout applicateur, cette tige étant obtenue par compression d'oxyde d'argent puis frittée afin de constituer un filtre de profondeur. Dans la pratique, la forme donnant les meilleurs résultats d'un point de vue des résultats microbiologiques est celle pour laquelle le rapport surface/volume est le plus grand possible. Le volume prédéterminé de substance médicamenteuse localisée dans le distributeur à proximité de l'orifice de l'embout applicateur est de préférence inférieur au volume d'une goutte. Le rapport entre ce volume prédéterminé mesuré en microlitres et la surface des moyens d'aseptisation mesurée en millimètres carrés est en tout cas inférieur à 1 et se situe de préférence en dessous de 0,5.

En résumé, grâce à la conception de l'ensemble du dispositif, de ses composants individuels et de leur interaction, on obtient un certain nombre d'avantages uniques à la fois pour le fabricant et l'utilisateur.

Au niveau de l'utilisation, étant donné que le piston ne peut qu'être poussé vers l'avant et ne peut en aucun cas être retiré vers l'arrière, une réaspiration d'air non stérile à l'intérieur du réservoir est totalement impossible. En effet, le poussoir est simplement adapté sur le piston sans être rendu solidaire de cet organe. Le filtre constitue une barrière aseptique très efficace et enfin les moyens d'aseptisation garantissent la stérilisation d' éventuelles gouttes de substance restant dans l'embout applicateur après un premier usage. De ce fait, la substance de traitement conserve sa stérilité au cours de l'utilisation du dispositif et on peut affirmer que grâce aux différents moyens énoncés ci-dessus il devrait être possible d'assurer un conditionnement aseptique de solutions ne pouvant pas être autoclavées sans recourir à l'utilisation d'un agent conservateur.

Le piston est en outre construit de telle manière, qu'après chaque usage, il supprime la pression résiduelle dans le dispositif, ce qui empêche un refoulement indésirable de substance médicamenteuse. Le problème de la contamination due à une pénétration de germes après un premier usage est résolu grâce à l'intervention d'un ou de plusieurs éléments ayant un effet oligodynamique. Cet effet produit par différents métaux lourds ou oxydes de métaux lourds tels que l'oxyde d'argent est bien connu. Pour qu'il soit efficace, il faut que la surface de contact de ces moyens d'aseptisation soit aussi grande que possible pour un volume de substance médicamenteuse à traiter aussi faible que possible. De ce fait, on garantit que le volume à traiter, c'est-à-dire le volume résiduel restant à proximité de l'orifice après chaque dépôt d'une goutte, est constant et qu'il est inférieur au volume d'une goutte. A cet effet également on prévoit d'utiliser de préférence un élément actif réalisé en argent et oxyde d'argent, fritté, se présentant sous la forme d'un filtre de profondeur pouvant en outre avoir une fonction de régulateur de débit.

## Revendications

1. Dispositif de stockage d'une substance médicamenteuse liquide et de distribution de gouttes ophtalmiques constituées par cette substance, comportant un réservoir cylindrique contenant ladite substance et un distributeur adapté à une extrémité de ce réservoir, ce distributeur étant pourvu d'un embout applicateur (14) équipé d'un orifice pour former et déposer lesdites gouttes dans l'oeil d'un patient, caractérisé en ce que le réservoir (10) est rigide et obturé, à l'extrémité sur laquelle est adapté le distributeur, par un dispositif régulateur de débit (19) comportant un organe régulateur dont l'ouverture est fonction d'une pression de seuil appliquée à ladite substance médicamenteuse liquide, en ce que ce réservoir est obturé à son autre extrémité par un piston coulissant (12) engagé dans ledit réservoir, ledit piston étant agencé pour générer ladite pression de seuil, en ce que l'organe régulateur de débit est associé à des moyens d'aseptisation ayant une action oligodynamique sur un volume prédéterminé de substance médicamenteuse localisée en amont dudit orifice, et en ce que ledit piston est pourvu de moyens élastiques agences pour absorber la pression résiduelle.

2. Dispositif selon la revendication 1, caractérisé en ce que le dispositif régulateur de débit (19) comporte au moins une membrane à micropores calibrés.

3. Dispositif selon la revendication 1, caractérisé en ce que le dispositif régulateur de débit (19) comporte au moins un orifice calibré.

4. Dispositif selon la revendication 1, caractérisé en ce que le dispositif régulateur de débit (19) comporte au moins un canal étranglé.

5. Dispositif selon la revendication 1, caractérisé en ce que le réservoir (10) comporte en outre un moyen d'obturation complémentaire amovible agencé pour empêcher le contact de la substance médicamenteuse avec le dispositif régulateur de débit pendant le stockage de cette substance.

6. Dispositif selon la revendication 5, caractérisé en ce que le moyen d'obturation complémentaire amovible est un bouchon d'obturation amovible (30, 50, 57) et en ce que le réservoir comporte une chambre de dérivation agencée pour loger au moins partiellement ce bouchon pendant l'utilisation de la substance médicamenteuse.

7. Dispositif selon la revendication 6, caractérisé en ce que le bouchon d'obturation amovible (30) comporte au moins un bourrelet annulaire (44) pour assurer l'étanchéité du réservoir pendant le stockage et au moins un évidement latéral (32) pour assurer l'écoulement de la substance médicamenteuse pendant l'utilisation, cet évidement étant agencé pour communiquer avec au moins un évidement ménagé à la base du réservoir.

8. Dispositif selon la revendication 7 caractérisé en ce que la hauteur de la chambre de dérivation est inférieure à la hauteur du bouchon d'obturation amovible.

9. Dispositif selon la revendication 6, caractérisé en ce que la hauteur de la chambre de dérivation est supérieure à la hauteur du bouchon d'obturation amovible.

10. Dispositif selon la revendication 5, caractérisé en ce que le bouchon d'obturation amovible (50) comporte une zone centrale d'épaisseur réduite (51) et en ce que le réservoir est équipé d'une aiguille (52) agencé pour percer ce bouchon dans sa zone centrale.

11. Dispositif selon la revendication 10, caractérisé en ce que ladite aiguille est fixe et montée à proximité du dispositif régulateur de débit (19).

12. Dispositif selon la revendication 10, caractérisé en ce que ladite aiguille est mobile et montée sur un support porte-aiguille mobile.

13. Dispositif selon la revendication 5, caractérisé en ce que le bouchon d'obturation amovible (57) comporte une soupape unidirectionnelle (58).

14. Dispositif selon les revendications 6 ou 7, caractérisé en ce que le bouchon d'obturation amovible (50, 57) et le piston mobile (12) comportent respectivement au moins un évidement et une protubérance de forme et de dimensions complémentaires.

15. Dispositif selon la revendication 1, caractérisé en ce que le dispositif régulateur de débit (19) est disposé à la base du distributeur (11).

16. Dispositif selon la revendication 15, caractérisé en ce que le dispositif régulateur de débit (19) est monté dans l'embout applicateur (14) du distributeur (11).

17. Dispositif selon la revendication 6, caractérisé en ce qu'il comporte au moins un évent (55) disposé à l'extrémité du réservoir (10) et débouchant dans la chambre de dérivation pour permettre l'évacuation de gaz contenus dans cette chambre avant l'utilisation de ce dispositif.

18. Dispositif selon la revendication 1, caractérisé en ce que le réservoir (10') comporte deux compartiments (10'a, 10'b) séparés par un bouchon intermédiaire mobile (72).

19. Dispositif selon la revendication 18, caractérisé en ce que l'un des compartiments (10'b) contient un solvant (71) et l'autre compartiment (10'a) contient une substance (70) destinée à être dissoute ou mélangée au solvant (71).

20. Dispositif selon la revendication 18, caractérisé en ce que le réservoir (10') comporte un renflement central (73) agencé pour permettre le passage du solvant liquide, lorsque le bouchon intermédiaire mobile (72) est amené au voisinage de ce renflement.

21. Dispositif selon la revendication 18, caractérisé en ce qu'il comporte un capuchon (74) de limitation de la course du poussoir (20) pour assurer le mélange des composants.

22. Dispositif selon la revendication 1, caractérisé en ce que le piston (12) comporte un plot de retenue rigide (12a) et une enveloppe souple (12b) définissant une cavité intérieure (12d), ce plot de retenue comportant un téton d'appui (12c), et en ce que la hauteur totale dudit plot avec le téton d'appui est sensiblement égale, au repos, à la hauteur de la cavité intérieure (12d).

23. Dispositif selon la revendication 1, caractérisé en ce que le piston (12) comporte un ressort spiral (21) agencé pour appliquer une force de poussée progressive.

24. Dispositif selon l'une quelconque des revendications précédentes, caractérisé en ce le réservoir (10) comporte une chemise cylindrique (40) qui constitue ses parois latérales.

25. Dispositif selon la revendication 1, caractérisé en ce que le réservoir cylindrique se compose d'un premier élément tubulaire (100) et d'un second élément tubulaire (101) qui s'emboîtent l'un dans l'autre, le premier étant obturé par un obturateur fixe (102) et par un piston mobile (104) et le second contenant un élément rigide (106) creux couplant mécaniquement le piston mobile (104) et le piston coulissant (105), ledit obturateur fixe (102) comportant une soupape unidirectionnelle permettant l'écoulement de la substance médicamenteuse vers le distributeur, et le fond dudit second élément tubulaire (101) étant ferme.

26. Dispositif selon la revendication 1, caractérisé en ce que ledit réservoir cylindrique (120) est obturé par un piston coulissant (121) solidaire d'un élément rigide creux (123) monté au fond d'une capsule (122) dans laquelle est emboîté partiellement ledit réservoir cylindrique, et en ce que ledit élément creux communique avec le distributeur à travers un filtre (125) et/ou lesdits moyens d'aseptisation.

27. Dispositif selon la revendication 1, caractérisé en ce que ledit organe régulateur de débit comporte un filtre de profondeur.

28. Dispositif selon la revendication 27, caractérisé en ce que ledit filtre de profondeur est constitué d'un matériau fritté contenant au moins un métal lourd ayant un effet oligodynamique antibactérien, choisi parmi les métaux lourds, les composés et les mélanges de ces métaux et les mélanges de ces composés.

29. Dispositif selon la revendication 1, caractérisé en ce que lesdits moyens d'aseptisation comportent de l'argent (24) et/ou de l'oxyde d'argent.

30. Dispositif selon la revendication 1, caractérisé en ce que ledit volume prédéterminé est inférieur au volume d'une goutte.

31. Dispositif selon la revendication 30, caractérisé en ce que le rapport entre le volume prédéterminé mesuré en microlitres et la surface desdits moyens d'aseptisation mesurée en millimètres carrés est inférieur à 1.

32. Dispositif selon la revendication 1, caractérisé en ce que lesdits moyens élastiques comportent une zone de moindre résistance ménagée du côté du piston en contact avec la substance médicamenteuse et agencée pour se déformer sous la poussée exercée sur ce piston et pour revenir dans son état normal à la fin de cette poussée.

## Claims

1. A device for storing a liquid medicinal substance and administering eye drops constituted by this substance, comprising a cylindrical reservoir containing the said substance and a distributor fitted on one end of this reservoir, this distributor being equipped with an applicator nozzle (14) provided with an orifice designed to form and to administer the said drops in the eye of a patient, characterized in that the reservoir (10) is rigid and sealed, at the end on which is fitted the distributor, by a flow rate controlling device (19) comprising a controlling element the opening of which is a function of the threshold pressure applied to the said liquid medicinal substance, in that this reservoir is sealed at its other end by a sliding piston (12) fitted into the said reservoir, the said piston being arranged such as to generate the said threshold pressure, in that the flow rate controlling device is connected to asepticizing means having an oligodynamic action on a predetermined volume of medicinal substance situated upstream from the said orifice, and in that the said piston is provided with elastic means arranged to absorb residual pressure.

2. A device in accordance with claim 1, characterized in that the flow rate controlling device (19) comprises at least one membrane with calibrated micropores.

3. A device in accordance with claim 1, characterized in that the flow rate controlling device (19) comprises at least one calibrated orifice.

4. A device in accordance with claim 1, characterized in that the flow rate controlling device (19) comprises at least one narrow channel.

5. A device in accordance with claim 1, characterized in that the reservoir (10) comprises in addition an additional movable sealing means arranged to prevent contact of the medicinal substance with the flow rate controlling device during storage of this substance.

6. A device in accordance with claim 5, characterized in that the additional movable sealing means is a movable sealing plug (30, 50, 57) and in that the reservoir comprises one bypass chamber arranged to accept at least partially this plug during use of the medicinal substance.

7. A device in accordance with claim 6, characterized in that movable sealing plug (30) comprises at least one ring shaped enlargement (44) to ensure impenetrability of the reservoir during storage and at least one lateral recess (32) to ensure the flow of the medicinal substance during use, this recess being arranged to communicate with at least one recess made in the base of the reservoir.

8. A device in accordance with claim 7, characterized in that the height of the bypass chamber is less than the height of the movable sealing plug.

9. A device in accordance with claim 6, characterized in that the height of the bypass chamber is greater than the height of the movable sealing plug.

10. A device in accordance with claim 5, characterized in that the movable sealing plug (50) comprises a thin central area (51) and in that the reservoir is fitted with a needle (52) arranged to pierce this plug in its central area.

11. A device in accordance with claim 10, characterized in that the said needle is fixed and mounted close to the flow rate controlling device (19).

12. A device in accordance with claim 10, characterized in that the said needle is mobile and mounted on a mobile needle carrier support.

13. A device in accordance with claim 5, characterized in that the movable sealing plug (57) comprises a unidirectional valve (58).

14. A device in accordance with claims 6 or 7, characterized in that the movable sealing plug (50, 57) and the mobile piston (12) comprise respectively at least one recess and one protuberance of complementary shape and dimensions.

15. A device in accordance with claim 1, characterized in that the flow rate controlling device (19) is arranged at the base of the distributor (11).

16. A device in accordance with claim 1, characterized in that the flow rate controlling device (19) is mounted in the applicator nozzle (14) of the distributor (11).

17. A device in accordance with claim 6, characterized in that it comprises at least one vent (55) arranged at the end of the reservoir (10) and leading onto the bypass chamber to allow evacuation of gases contained in this chamber before using the device.

18. A device in accordance with claim 1, characterized in that the reservoir (10') comprises two compartments (10'a, 10'b) separated by an intermediate mobile plug (72).

19. A device in accordance with claim 18, characterized in that the one of the compartments (10'b) contains a solvent (71) and the other compartment (10'a) contains a substance (70) designed to be dissolved or mixed with the solvent (71).

20. A device in accordance with claim 18, characterized in that the reservoir (10') comprises a central enlargement (73) arranged to enable the passage of the liquid solvent, when the intermediate mobile plug (72) is brought close to this enlargement.

21. A device in accordance with claim 18, characterized in that it comprises a hood (74) to limit the stroke of the plunger (20) to mix the components.

22. A device in accordance with claim 1, characterized in that the piston (12) comprises a rigid retention stop (12a) and a flexible sheath (12b) defining an internal cavity (12d), this retention stop comprising a supporting stud (12c), and in that the total height of the said stop with the supporting stud is roughly equal, at rest, to the height of the internal cavity (12d).

23. A device in accordance with claim 1, characterized in that the piston (12) comprises a spiral spring (21) arranged to apply a gradual thrust.

24. A device in accordance with any one of the preceding claims, characterized in that the reservoir (10) comprises a cylindrical jacket (40) which constitutes its side walls.

25. A device in accordance with claim 1, characterized in that the cylindrical reservoir is made up of a first tubular element (100) and a second tubular element (101) which fit into each other, the first being sealed by a fixed seal (102) and by a mobile piston (104) and the second containing a hollow rigid element (106) mechanically connecting the mobile piston (104) and the sliding piston (105), the said fixed seal (102) comprising a unidirectional valve enabling the medicinal substance to flow towards the distributor, and the bottom of the said second tubular element (101) being closed.

26. A device in accordance with claim 1, characterized in that the said cylindrical reservoir (120) is sealed by a sliding piston (121) solid with a hollow rigid element (123) mounted on the bottom of a capsule (122) in which there is partially inserted the said cylindrical reservoir, and in that the said hollow element communicates with the distributor via a filter (125) and/or the said asepticizing means.

27. A device in accordance with claim 1, characterized in that the said flow rate controlling device comprises a depth filter.

28. A device in accordance with claim 27, characterized in that the said depth filter is made up of a sintered material containing at least one heavy metal having an oligodynamic anti-bacterial effect, chosen from among the heavy metals, compounds and mixtures of these metals and mixtures of these compounds.

29. A device in accordance with claim 1, characterized in that the said asepticizing means contain silver (24) and/or silver oxide.

30. A device in accordance with claim 1, characterized in that the said predetermined volume is less than the volume of one drop.

31. A device in accordance with claim 30, characterized in that the ratio between the predetermined volume measured in microlitres and the surface area of the said asepticizing means measured in square millimetres is less than 1.

32. A device in accordance with claim 1, characterized in that the said elastic means contain a less resistant area installed on the piston side in contact with the medicinal substance and arranged to change shape under the thrust exerted on this piston and to return to its normal state at the end of this thrust.

## Patentansprüche

1. Vorrichtung zum Aufbewahren einer flüssigen medizinischen Substanz und zur Ausgabe von Augentropfen, die aus der medizinischen Substanz gebildet sind, bestehend aus einem zylindrischen Behälter, der die Substanz enthält, und aus einem Verteilstück, das an einem Ende des Behälters angebracht ist, wobei das Verteilstück mit einem Ansatz (14) versehen ist, der eine Öffnung für die Bildung von Tropfen und das Einbringen der Tropfen in das Auge eines Patienten aufweist,
**dadurch gekennzeichnet,** daß
der Behälter (10) starr ist und an dem Ende, das mit dem Verteilstück versehen ist, von einer Einrichtung (19) für die Einstellung des Durchsatzes verschlossen ist, die ein Regelorgan besitzt, dessen Öffnung von einem Druck abhängt, der auf die flüssige medizinische Substanz ausgeübt wird, daß der Behälter an seinem anderen Ende von einem gleitenden Kolben (12) verschlossen ist, der in den Behälter eingeführt ist, wobei der Kolben zum Aufbringen des Druckes auf die medizinische Substanz vorgesehen ist, daß das Organ für die Einstellung des Durchsatzes mit Desinfizierungs-Einrichtungen verbunden ist, die eine geringe dynamische Wirkung auf eine vorher bestimmte Menge der medizinischen Substanz vor der Öffnung haben, und daß der Kolben mit elastischen Einrichtungen versehen ist, die zur Aufnahme des restlichen Druckes eingerichtet sind.

2. Vorrichtung nach Anspruch 1,
**dadurch gekennzeichnet,** daß
die Einrichtung (19) für die Einstellung des Durchsatzes mindestens eine Membrane mit kalibrierten Mikroporen enthält.

3. Vorrichtung nach Anspruch 1,
**dadurch gekennzeichnet,** daß
die Einrichtung (19) für die Einstellung des Durchsatzes mindestens eine kalibrierte Öffnung aufweist.

4. Vorrichtung nach Anspruch 1,
**dadurch gekennzeichnet,** daß
die Einrichtung (19) für die Einstellung des Durchsatzes mindestens einen mit einer Engstelle versehenen Kanal besitzt.

5. Vorrichtung nach Anspruch 1,
**dadurch gekennzeichnet,** daß
der Behälter (10) außerdem eine abnehmbare zusätzliche Verschlußeinrichtung enthält, die vorgesehen ist, um die Berührung der medizinischen Substanz mit der Einrichtung für die Einstellung des Durchsatzes während der Aufbewahrung der Substanz zu verhindern.

6. Vorrichtung nach Anspruch 5,
**dadurch gekennzeichnet,** daß
die abnehmbare zusätzliche Verschlußeinrichtung ein abnehmbarer Verschlußstopfen (30,50,57) ist, und daß der Behälter eine Umlaufkammer besitzt, die zur mindestens teilweisen Aufnahme des Stopfens während des Gebrauchs der medizinischen Substanz eingerichtet ist.

7. Vorrichtung nach Anspruch 6,
**dadurch gekennzeichnet,** daß
der abnehmbare Verschlußstopfen (30) mindestens einen ringförmigen Wulst (44) für die Sicherstellung der Abdichtung des Behälters während der Aufbewahrung und mindestens eine seitliche Aussparung (32) für die Sicherstellung des Abflusses der medizinischen Substanz während des Gebrauchs aufweist, wobei die Aussparung mit mindestens einer Aussparung im unteren Bereich des Behälters verbindbar ist.

8. Vorrichtung nach Anspruch 7,
**dadurch gekennzeichnet,** daß
die Höhe der Umlaufkammer kleiner als die Höhe des abnehmbaren Verschlußstopfens ist.

9. Vorrichtung nach Anspruch 6,
**dadurch gekennzeichnet,** daß
die Höhe der Umlaufkammer größer als die Höhe des abnehmbaren Verschlußstopfens ist.

10. Vorrichtung nach Anspruch 5,
**dadurch gekennzeichnet,** daß
der abnehmbare Verschlußstopfen (50) einen mittleren Bereich von geringer Dicke (51) aufweist, und daß der Behälter mit einer Nadel (52) zum Durchbohren des Stopfens in seinem mittleren Bereich versehen ist.

11. Vorrichtung nach Anspruch 10,
**dadurch gekennzeichnet,** daß
die Nadel feststehend und in der Nähe der Einrichtung (19) für die Einstellung des Durchsatzes angeordnet ist.

12. Vorrichtung nach Anspruch 10,
**dadurch gekennzeichnet,** daß
die Nadel beweglich und auf einem beweglichen Nadelhalter angeordnet ist.

13. Vorrichtung nach Anspruch 5,
**dadurch gekennzeichnet,** daß
der abnehmbare Verschlußstopfen (57) ein Rückschlagventil (58) enthält.

14. Vorrichtung nach Anspruch 6 oder 7,
**dadurch gekennzeichnet,** daß
der abnehmbare Verschlußstopfen (50,57) und der bewegliche Kolben (12) jeweils mindestens eine Aussparung und einen Vorsprung mit komplementären Formen und Abmessungen aufweisen.

15. Vorrichtung nach Anspruch 1,
**dadurch gekennzeichnet,** daß
die Einrichtung (19) für die Einstellung des Durchsatzes unten am Verteilstück (11) angeordnet ist.

16. Vorrichtung nach Anspruch 15,
**dadurch gekennzeichnet,** daß
die Einrichtung (19) für die Einstellung des Durchsatzes in dem Ansatz (14) des Verteilstückes (11) eingebaut ist.

17. Vorrichtung nach Anspruch 6,
**gekennzeichnet durch**
mindestens einen Entlüfter (55), der am Ende des Behälters (10) angeordnet ist und in die Umlaufkammer einmündet, um vor dem Gebrauch der Vorrichtung eine Entlüftung der Gase, die in der Umlaufkammer enthalten sind, zu ermöglichen.

18. Vorrichtung nach Anspruch 1,
**dadurch gekennzeichnet,** daß
der Behälter (10') zwei Abteile (10'a,10'b) besitzt, die durch einen beweglichen Zwischenstopfen (72) voneinander getrennt sind.

19. Vorrichtung nach Anspruch 18,
**dadurch gekennzeichnet,** daß
das eine Abteil (10'b) ein Lösungsmittel (71) und das andere Abteil (10'a) eine Substanz (70) enthält, die dazu bestimmt ist, aufgelöst oder mit dem Lösungsmittel (71) vermischt zu werden.

20. Vorrichtung nach Anspruch 18,
**dadurch gekennzeichnet,** daß
der Behälter (10') einen mittleren Wulst (73) aufweist, der für den Durchfluß des flüssigen Lösungsmittels ausgelegt ist, wenn der bewegliche Zwischenstopfen (72) in die Nähe des Wulstes gebracht ist.

21. Vorrichtung nach Anspruch 18,
**gekennzeichnet durch**
eine Kappe (74) für die Begrenzung des Verstellweges des Stempels (20), damit die Vermischung der Bestandteile sichergestellt ist.

22. Vorrichtung nach Anspruch 1,
**dadurch gekennzeichnet,** daß
der Kolben (12) einen starren Haltestöpsel (12a) und eine biegsame Ummantelung (12b), die einen inneren Hohlraum (12d) bildet, aufweist, wobei der Haltestöpsel eine Abstützung (12c) besitzt, und daß die gesamte Höhe des Haltestöpsels mit der Abstützung in der Ruhestellung im wesentlichen der Höhe des inneren Hohlraumes (12d) entspricht.

23. Vorrichtung nach Anspruch 1,
**dadurch gekennzeichnet,** daß
der Kolben (12) eine Spiralfeder (21) besitzt, die für die Ausübung einer progressiven Schubkraft ausgelegt ist.

24. Vorrichtung nach einem der vorhergehenden Ansprüche,
**dadurch gekennzeichnet,** daß
der Behälter (10) einen zylindrischen Mantel (40) aufweist, der seine Seitenflächen bildet.

25. Vorrichtung nach Anspruch 1,
**dadurch gekennzeichnet,** daß
der zylindrische Behälter aus einem ersten Rohrstück (100) und aus einem zweiten Rohrstück (101) besteht, die ineinandergesteckt sind, wobei das erste Rohrstück durch einen feststehenden Verschluß (102) und einen beweglichen Kolben (104) verschlossen ist und das zweite Rohrstück einen starren hohlen Teil (106) enthält, der den beweglichen Kolben (104) mit dem gleitenden Kolben (105) mechanisch koppelt, wobei der feststehende Verschluß (102) ein Rückschlagventil für den Abfluß der medizinischen Substanz zu dem Verteilstück hin enthält, und wobei der Boden des zweiten Rohrstückes (101) verschlossen ist.

26. Vorrichtung nach Anspruch 1,
**dadurch gekennzeichnet,** daß
der zylindrische Behälter (120) durch einen gleitenden Kolben (121) verschlossen ist, der mit einem starren hohlen Teil (123) fest verbunden ist, welcher am Boden einer Kapsel (122) eingebaut ist, in die der zylindrische Behälter teilweise gesteckt ist, und daß der starre hohle Teil über einen Filter (125) und/oder über die Desinfektions-Einrichtungen mit dem Verteilstück in Verbindung steht.

27. Vorrichtung nach Anspruch 1,
**dadurch gekennzeichnet,** daß
die Einrichtung (19) für die Einstellung des Durchsatzes einen Tiefbettfilter enthält.

28. Vorrichtung nach Anspruch 27,
**dadurch gekennzeichnet,** daß
der Tiefbettfilter aus einem Sinterwerkstoff besteht, der mindestens ein Schwermetall enthält, das eine geringe dynamische antibakterielle Wirkung besitzt und aus den Schwermetallen, den Verbindungen und den Gemischen dieser Schwermetalle und den Gemischen dieser Verbindungen ausgewählt ist.

29. Vorrichtung nach Anspruch 1,
**dadurch gekennzeichnet,** daß
die Desinfektions-Einrichtungen Silber (24) und/oder Silberoxyd enthalten.

30. Vorrichtung nach Anspruch 1,
**dadurch gekennzeichnet,** daß
die vorherbestimmte Menge kleiner als das Volumen eines Tropfens ist.

31. Vorrichtung nach Anspruch 30,
**dadurch gekennzeichnet,** daß
das Verhältnis zwischen der vorherbestimmten Menge, gemessen in Mikrolitern, und der Fläche der Desinfektions-Einrichtungen, gemessen in Quadratmillimetern, kleiner als 1 ist.

32. Vorrichtung nach Anspruch 1,
**dadurch gekennzeichnet,** daß
die elastischen Einrichtungen einen Bereich von geringer Festigkeit aufweisen, der auf der Seite des Kolbens, der mit der medizinischen Substanz in Berührung kommt, vorgesehen und ausgelegt ist, um sich unter der Schubkraft, die auf den Kolben ausgeübt wird, zu verformen und nach dem Ausbleiben dieser Schubkraft wieder einen normalen Zustand zu erlangen.
